# EUROPEAN PATENT APPLICATION

(11) **EP 4 692 797 A1**
(43) Date of publication of application: **11.02.2026**
(21) Application number: 24813681.4
(22) Date of filing: 08.01.2024
(51) Int. Cl.: G01N 33/543, G01N 33/53

(54) **DEVICE FOR DETECTING ALLERGEN ANTIBODIES IN LIQUID SAMPLE AND METHOD**

(30) Priority: 29.05.2023 CN 202310616287; 04.01.2024 CN 202410014572
(71) Applicant: He, Lujiang, Hangzhou, Zhejiang 311100 (CN)
(72) Inventor: WANG, Shuming, Hangzhou, Zhejiang 310030 (CN); SHEN, Meiyan, Hangzhou, Zhejiang 310030 (CN); GONG, Wange, Hangzhou, Zhejiang 310030 (CN); SHI, Shumin, Hangzhou, Zhejiang 310030 (CN); WANG, Haiqing, Hangzhou, Zhejiang 310030 (CN); WANG, Haimeng, Hangzhou, Zhejiang 310030 (CN)
(74) Representative: Loo, Chi Ching
(86) International application number: PCT/CN2024/071094
(87) International publication number: WO 2024/244475

(57) **Abstract**

The present invention provides a device for testing an allergen antibody (IgE) in a liquid sample comprising a testing area and a water absorption area, where the testing area is used to contact with liquid, and the water absorption area is located downstream of the testing area and used to absorb the liquid from the testing area; a plurality of first receptors immobilized onto the testing area, and each of the first receptors is capable to specifically bind to a plurality of different allergen antibodies in the liquid sample. Such a device can be used to once test the presence or absence or level of a plurality of allergen antibodies in the liquid sample, thereby achieving an efficient and convenient testing effect.

## Description

### CROSS-REFERENCE TO RELATED APPLICATIONS

This application claims priority to Chinese Patent Applications, Application No.: 202310616287X, filed on May 29, 2023, Application No.:2024100184600, filed on January 04, 2024; Application No.2024100145729, filed on January 04, 2024; and all disclosures of this application, including but not limited to the abstract, claims, accompanying drawings, and specification of this application are incorporated by reference in their entirety as a part of this application.

### BACKGROUND OF THE INVENTION

### Field of the Invention

The present invention relates to the field of in vitro diagnosis and detection, and in particular, to an immune reaction chip for testing an analyte in a liquid sample based on lateral flow and an application method thereof.

### Description of the Related Art

Immunological analysis methods are widely used in the detection of trace substances because of their unique specificity, high sensitivity and practicality. Such methods are widely used in medicine, veterinary medicine, agriculture, animal husbandry, food safety, environmental monitoring, biological safety and other fields.

Based on the principle of specific binding of antigens and antibodies, multiple substances in a same sample can be simultaneously tested. For example, solid protein chip, liquid protein chip, membrane strip immunoblotting, microarray enzyme-linked immunosorbent assay, and dot ELISA. All these methods have the characteristics of less samples and high throughput, and are used in various applications, such as allergen detection, detection of autoimmune diseases, as well as detection of a plurality of tumor labels, pathogens, pesticide and veterinary drug residues and a plurality of natural toxins.

Up to now, all immunological testing methods require that immune reaction antigens or antibodies are immobilized on a solid-phase surface, such as flat glass surface, polymer flat surface, polymer microsphere surface and membrane structure. Some of the testing methods are based on membrane materials and can be used to simultaneously detect a plurality of analytes. Membrane materials are essentially porous materials formed by interlacing multi-branched fibers together, and inherently have the properties of instantaneous binding of a large number of proteins, chemical inertness, and capillary action, such that immunological detection methods based on membrane materials are diverse and widely applied. At present, dot enzyme-linked immunosorbent (Dot-ELISA), Western Blot, flow through and lateral flow are main application methods.

All immune reactions need to be carried out in a water-based liquid. In the liquid, antigens antibodies give rise to specific binding through collision (immune reaction), finally forming an immune complex. When membranes are used as solid-phase carriers, immune reaction mainly occurs at an interface between a solid phase and a liquid phase. The effectiveness of the immune reaction is affected by not only the concentrations of immune reaction antigens or antibodies in the liquid phase, the distribution density of immune reaction antigens or antibodies on the surface of the solid phase and their affinity, but also the effective contact at the interface between a solid phase and a liquid phase.

In practical applications, a testing method needs to meet the requirements of high sensitivity, stable repeatability (precision), simplicity and rapidity. Therefore, it is necessary to make a reasonable design according to the principle of immune reaction as mentioned above. However, there are some inherent defects in the current main methods.

For Dot-ELISA and Western Blot, membranes are used as solid carriers, mainly playing the role of immobilizing antigens or antibodies. When the liquid is added to the membrane, the main reaction between the solid phase and the liquid phase occurs at the contact surface between the membrane surface and the liquid, while the effective contact between the inner surface of the membrane and the liquid is relatively weak. Such relatively weak contact makes the antigens or antibodies immobilized inside the membrane unable to react with the corresponding antibodies or antigens in the liquid phase quickly. Therefore, in practical applications, a long incubation time is needed to give rise to sufficient immune reaction between the antigens and the antibodies and is usually more than 30 minutes, and additional shaking and heating are needed to increase the migration rate of the antigens or antibodies in the liquid phase. This way of prolonging the incubation time and additionally increasing an external force and an environmental temperature due to inefficient collision also results in increasing the risk of nonspecific binding. At present, a common solution is to enhance the washing intensity after immune reaction, such as prolonged immersion in the washing liquid, oscillation support, and repeatedly washing several times, for example, immersion for 5 minutes and repeatedly washing for more than 3 times. This in turn reduces the convenience of the testing method. At present, it takes several hours to apply the testing methods in this mode, and a variety of auxiliary measures and complex operations are introduced in the process to obtain results. For example, the testing methods should be realized with the support of some equipment (such as heating equipment and mechanical vibration equipment).

For flow through, the membrane is also used as a solid-phase material to immobilize an immune element, and the liquid used in each step of the immune reaction flows through the membrane in the direction perpendicular to the plane of the membrane. The percolation of the analyte in the liquid is driven by the gravity of the liquid and the capillary action of the absorbent material located on the lower layer of the membrane. After percolation, the analyte can be captured by the antigens or antibodies immobilized on the membrane, followed by dropwise addition of labeled antigens or antibodies, and driven by the gravity of the liquid and the capillary action of the absorbent material on the lower layer of the membrane. The labeled antigens or antibodies bind to the captured analyte to form an immune complex. A membrane with a small pore size, such as 0.45µm or 0.22µm nitrocellulose membrane, is used in the method, to increase the density of immobilized antigens or antibodies; however, it is difficult to ensure the effective formation of the immune complex because of the very fast percolation rate and the short collision time for the immune reaction between immobilized antigens or antibodies, analytes and labeled antigens or antibodies. This leads to the low sensitivity of the method. In addition, the rate of the liquid flowing through the membrane will also be affected by the close adhesion between the absorbent material and the membrane, such that the repeatability of the formation amount of the immune complex is poor. The method is limited in its application.

For lateral flow, because the membrane has the porous property, capillary diffusion serves as the driving force when the membrane contacts with the liquid phase; in this case, the liquid including the sample and the labeled antigens or antibodies can flow through the immobilized and paired antigens or antibodies in the testing area for immune reaction; and then the percolated liquid is continuously absorbed by the absorbent material; the absorbent material serves as the driving force, such that all liquids finally flow through the testing area to immobilize the antigens or antibodies; the labels will accumulate in the testing area due to the formation of the immune complex; and the accumulated labels are related to the presence or absence or quantity of the analytes. Labels used include colloidal gold, latex particles, fluorescent microspheres, quantum dots and the like. At present, all labeling techniques are used to test a limited amount of analytes in lateral flow. In other words, a small amount of analytes are tested, and no more than three analytes are tested by one reagent strip. The fundamental reason is that the amplification mode of the immune complex used in the mode substantially belongs to physical amplification. In order to improve the testing sensitivity, at present, the size of all the labels is larger than the molecular scale of the labeled object (for example, the antibody molecule in the liquid is about 6nm, while the colloidal gold as the label is usually over 40nm, and the latex beads are 200nm), and the testing area through which the liquid flows subsequently is affected by the accumulation degree of the labels in the testing area through which the liquid flows first. Usually, no more than three kinds of analytes are tested by a lateral flow device. This is mainly due to the fact that in addition to the influence induced by the above items, the total amount of labels carried in the device is limited due to the relatively large microscopic size of the labels. At present, a common solution is that test strips capable to test one or a small number of analytes are respectively prepared, and then the test strips are assembled into a plastic clamping slot, for example, for testing abuse of drugs in urine. Essentially, the solution is to combine a plurality of detection reagents. This requires doubled sample sizes, so only a small number of analytes can be measured.

When the existing immune reaction chips based on porous membrane materials are used to measure a plurality of testing indicators, a plurality of independent measurement sites are selected, and the test liquid is dripped to each of the independent measurement sites for immune reaction. There are some problems in this way: when excessive testing indicators are measured, the operation is complicated and the sample size is large; moreover, when the concentration of the analytes in the sample is too high or too low, the measurement result may be inaccurate.

Therefore, it is urgent to find an immune reaction detection method based on porous membrane materials, and the detection method meets the requirements of simplicity, rapidity, high sensitivity and stable repeatability (precision) and is applied to the simultaneous analysis of a plurality of analytes.

### BRIEF SUMMARY OF THE INVENTION

In order to solve the problems existing in the conventional technology, the present invention provides a chip element suitable for testing an analyte in a liquid sample with biomaterials and an application method thereof. The method provided by the present invention can be used to simultaneously test a plurality of analytes and is easy to operate, and can be suitable for home self-testing such as OCT and testing by professional organizations. In addition, the quantity of analytes to be tested may be more than three and the required sample size is very small; the method is especially suitable for a case that 10-100 different analytes need to be tested once; in a case that the sample size is limited, the testing device and method of the present invention are particularly effective.

The present invention provides a testing device for testing an analyte in a liquid sample, and the device includes a testing area and a liquid absorption area that is in fluid communication with the testing area. The liquid absorption area can be used to absorb a large amount of liquid from the testing area for many times, such that different liquids flow through the testing area to complete the assay or test of the analyte.

In some embodiments, the testing area is provided or arranged on a porous material having a water absorption property; and the water absorption property is characterized in that liquid absorption or liquid flowing on the porous material mainly depends on the capillary action. In some embodiments, after the porous material itself is moisturized or when the porous material itself loses the capillary force thereof, if it is desired that other different types of liquids are allowed to continue flowing on the porous material, the different types of liquids are allowed to flow through the porous material and the testing area under the capillary force of the water absorption area, thereby completing the test of the analyte. The diffusion rate of the liquid can be accurately controlled. In some embodiments, the diffusion direction of the liquid on the porous material can be controlled; in some embodiments, the flowing direction of the liquid on the porous membrane is unidirectional; and a plurality of liquids flow in a single same direction at different time.

The present invention overcomes defects in the prior art from three aspects: the formation principle of an immune complex, the characteristics of a solid-liquid interface between a fibrous membrane and a liquid, and a way of transforming the formed immune complex into an observable signal. Meanwhile, an immune reaction chip element with unidirectional lateral diffusion and an application method thereof are designed. The immune reaction chip and the application method thereof have advantages of rapidity, simplicity, high sensitivity and good repeatability, and provide the possibility of qualitative and quantitative analysis for simultaneous detection of a plurality of indicators. The method is suitable for not only manual operation but also automatic analysis with simple apparatuses. The immune reaction chip here has the same meaning as the "device" described in the present invention.

In some embodiments, a plurality of testing units are arranged on the testing area on the porous membrane; each testing unit is used to test one analyte; alternatively, the plurality of testing units can be used to test a plurality of different analytes. In some embodiments, the plurality of testing units are distributed on the porous membrane in an array, and the testing units form one testing area. In some embodiments, one first receptor is immobilized onto each testing area and specifically captures only a specific analyte in the liquid sample, such as an antibody or an antigen. In some embodiments, the receptor can bind to another binding element, for example, biotin; however, the receptor can only bind to one analyte in the liquid sample. When one first receptor is immobilized onto each of the testing units, an array for a plurality of first receptors is formed and each of the first receptors can specifically bind to the analyte in the liquid sample, such that the plurality of analytes in the same liquid sample can be tested. In the test, the liquid sample flows on the porous membrane depending on the porous membrane itself or the cooperation of the porous membrane with the water absorption area. Such flow is lateral flow in a same direction. In some embodiments, in addition to the liquid sample itself, other kinds of liquids can also be allowed to flow on the porous membrane and are involved in an entire testing process, to finally determine the presence or absence of quantity of the analytes. In some embodiments, a plurality of immobilized binding sites are arranged inside the porous membrane; for example, the binding sites are used to immobilize the plurality of first receptors, can be arranged side by side or back and forth, or form an array, without affecting the detection of the binding sites. Therefore, the liquid sample can be added once, and the test liquid sequentially flows through all binding sites, thereby generating multiple testing indicators. In some embodiments, the testing units can be arranged on the testing area in any form, or spaced from each other, or arranged in any pattern, for example, circle, square, trapezoid, and rhombus.

In some embodiments, in addition to the liquid sample, other liquids are further included, for example, including but not limited to, one or more washing liquids, a blocking liquid including a blocking reagent, a solution including second receptor, and a reaction liquid capable to react with a label. The washing liquids may be washing liquids with various purposes; for example, after the liquid sample flows through the porous membrane, the washing liquids are allowed to pass through the porous membrane to remove the residual liquid sample, residual impurities, or the like. In some embodiments, after the solution including second receptor passes through the porous membrane, another washing liquid can pass through the porous membrane to remove the residual solution including second receptor. In some embodiments, the liquids may also be a plurality of washing liquids and a plurality of solutions including second receptor. In some embodiments, the liquids may also be the reaction liquid; the reaction liquid includes the reaction reagent and reacts with the label on the immune complex immobilized on the testing unit to generate a signal substance; after the signal substance is generated, the washing liquids still can be used for washing to remove the residual substance. The liquids include, but are not limited to, the liquid sample, a mixture of the liquid sample and a liquid sample diluent, the washing liquid, an enzyme-labeled antigen or antibody liquid, a small molecule labeled antigen or antibody liquid, an enzyme-labeled ligand liquid specifically binding to small molecules, the reaction liquid, the blocking liquid, and a combination or mixture of the above liquids. In some embodiments, the different liquids are in advance stored in a multi-chamber structure; when the liquids need to perform reaction, the end of the testing device for receiving the liquid is inserted into a chamber to contact the different liquids. Of course, automatic operation can also be adopted, such that the test strip can be automatically inserted into various chambers to contact with the different liquids.

Of course, the blocking liquid is used to block blank areas on the porous membrane or blank areas on the testing unit to reduce false positives. In some embodiments, the blocking liquid includes the blocking reagent; and the blocking reagent is mainly used to block several sites on the porous membrane not to bind to the second receptor. The sites can be the ones in the blank areas or on the testing unit. In some embodiments, the second receptor in the solution including second receptor specifically binds to the analyte captured by the first receptor. Specific binding here means that the binding efficiency of the second receptor to the analyte is higher than that of the second receptor to other substances. For example, if the binding efficiency of a complex formed by the first receptor and the analyte to the second receptor is 100%, the second receptor completely (100%) cannot bind to other substances, for example, other complexes and other analytes. If the binding efficiency ranges from 95%-99%, the binding efficiency of the second receptor specifically binding to the complex formed by the first receptor and the analyte ranges from 95%-99%, and the second receptor is only 5%-1% likely to bind to other substances, for example, other complexes and other analytes. Here, the specific binding efficiency may be 100% or may be more than 60%.

In some embodiments, the second receptor can specifically bind to the analyte. In some embodiments, the second receptor can specifically bind to specific sites on the complex, for example, the second receptor can specifically bind to the first receptor, or the first receptor can bind to the analyte to form partial sites on the complex. In some embodiments, the first receptor can specifically bind to the antibody in the liquid sample if being the antigen, and the second receptor can be the antibody of the antigen in the liquid sample, or other similar receptors capable to specifically bind to the antibody in the liquid sample. For example, the first receptor can be a first antibody of the antigen in the liquid sample, and the second receptor can be a second antibody of the antigen in the liquid sample. If the antigen in the liquid sample needs to be tested, the first receptor can be the first antibody of the antigen, and the second receptor can be the second antibody of the antigen.

In some embodiments, the second receptor also includes the label that can generate a signal with the reaction reagent in the reaction liquid, and the presence or absence of the signal indicates the quantity or presence or absence of the analytes. In some embodiments, the label does not need to contact with the reaction reagent, but directly carries an intuitive signal, for example, colored particles, colored dyes, or fluorescent dyes. The testing results of the colored particles can be directly read with naked eyes; and if the fluorescent dyes are used, the testing results of the fluorescent dyes can be read by equipment capable to read fluorescence. For example, the label can also be gold particles, latex particles or water-soluble dyes. In some embodiments, washing liquids or blocking liquids are also needed when a label with an intuitive symbol is used. The washing liquids or blocking liquids can be used to clean the porous membrane after the liquid sample flows through the porous membrane, or the porous membrane can be cleaned after the blocking liquids flow through the porous membrane, or after the second receptor including a label flows through the porous membrane.

When the label needs to react with the reaction liquid to generate a signal, the label itself cannot directly carry the signal, but reacts with the reaction reagent to generate the signal. Such "reaction" can mean chemical reaction, or can mean that the reaction reagent contacts with the label to change the spatial concept and generate the signal. The signal can be read by the naked eyes or by equipment. The signal may be a color change signal, a light signal, an electricity signal, a ray signal and other signals. The color signal may denote color appearance or disappearance; the light signal may denote a luminescent substance and change in light wave or wavelength; the electricity signal may denote a direct current or alternating current signal; and the ray signal may denote electron emergence or electron flow.

In some embodiments, the label is an enzyme, and the reaction reagent in the reaction liquid contacts with the enzyme to generate colored substances; the colored substances are gathered on the testing unit, thus indicating the presence or absence or quantity of the analytes on the testing unit.

In some embodiments, the label is an enzyme, and the reaction reagent in the reaction liquid contacts with the enzyme to generate fluorescent substances; the fluorescent substances are gathered on the testing unit, thus indicating the presence or absence or quantity of the analytes on the testing unit.

In some embodiments, to enable the porous membrane to contact with the liquid conveniently, the liquid is allowed to flow on the porous membrane; an area is arranged on the porous membrane and used to contact with the liquid, for example, an area is provided upstream of the testing area and used to contact with the liquid, and the water absorption area is provided downstream of the testing area and used to attract the liquid on the porous membrane to flow, or the water absorption area continuously provides the capillary force of the porous membrane, such that different liquids can flow from the upstream of the testing area to the downstream of the testing area for many times. It can be understood that a same porous membrane is divided into several areas, where one area is used to receive the liquid, the other area contacts with the water absorption area, and the testing area is set between these two areas. The area for receiving the liquid, the area contacting with the water absorption area, and the testing area are all located on the porous membrane made of a same material. Of course, it is also possible that porous membranes made of different materials are used for these three areas. For example, the testing area is located on the porous membrane, and the area for receiving the liquid can be another porous membrane not used for the testing area.

In some embodiments, a porous membrane with a specific pore size is selected. When a test liquid gives rise to immune reaction with the binding site, the immune complex is generated at an immobilizing site. A porous membrane with a large pore size is used to ensure that the immune complex will not block a membrane channel and prevent the unreacted test liquid from continuing to diffuse towards the liquid absorption area and reacting with a posterior binding site. A plurality of testing units are arranged on the porous membrane in an array, and the testing units always contact with the liquid successively in a liquid flowing direction. If the testing units contacting with the liquid firstly react with the liquid, the membrane is blocked, and a liquid flow rate slows down or the liquid flowing direction is changed, the testing units contacting with the liquid later incompletely react with the liquid and some of the testing units may not contact with the liquid, such that a part of the testing units can be effectively tested and the other part of the testing units cannot be effectively tested.

In some embodiments, the liquid is allowed to flow through the porous membrane as much as possible to minimize the liquid flowing on the surface of the porous membrane, which may cause a "torrent" and affect the testing results. Therefore, in some embodiments, a liquid blocking element is provided downstream of the area for receiving the liquid to prevent the liquid from flowing above the surface of the porous membrane and to allow the liquid to flow through the porous membrane, such that the liquid can fully contact with each testing unit. In some embodiments, the binding site being mainly immobilized inside the porous membrane can effectively prevent the liquid reagent from flowing h above the surface of the porous membrane, so the liquid concentrates on a capillary channel inside the porous membrane to diffuse, which can increase the formation efficiency of immune complexes at the binding site and increase the accuracy and sensitivity of test.

In some embodiments, the liquid blocking element can also be provided downstream of the testing area to reduce the liquid flow rate, allow the liquid to stay on the porous membrane for more time, and also allow the liquid to flow through the porous membrane to reach the water absorption area as far as possible, such that the water absorption area can absorb the liquid from the porous membrane under the capillary action as much as possible to avoid liquid flowing from the surface of the porous membrane to the water absorption area.

In some embodiments, the liquid blocking element is respectively provided downstream of the area for receiving the liquid, the upstream of testing area, the downstream of the testing area and the upstream of the water absorption area; thus, on the one hand, the liquid is allowed to flow through the porous membrane as much as possible to reduce the liquid flowing above the surface of the porous membrane; on the other hand, the liquid is allowed to stay on the porous membrane for more time and contact with a plurality of testing units. This is particularly important when a plurality of analytes are tested once. When the plurality of testing units are provided, for example, when 10-100 different analytes are tested, the testing units contact with the liquid successively, and the liquid is allowed to flow through the porous membrane and is more likely to contact with the testing units, thereby improving the testing efficiency.

In other some embodiments, one end of the porous membrane is used to receive the liquid, and the liquid flows through the testing area on the porous membrane and finally flows into the downstream water absorption area. For example, these liquids can be liquid samples, washing liquids, second receptors including a label, reaction liquids, and the like. However, in some cases, different testing units are tested only depending on the time when the liquid flows through the porous membrane. Although such test can be conducted, the testing effect is not desired. It is hoped that the liquid will stay on the porous membrane and fully contact with the testing units for full reaction. Therefore, the liquid blocking element of the present invention, especially for the liquid blocking element provided downstream of the testing area, can allow the liquid to stay on the porous membrane more, instead of flowing into the water absorption area quickly.

In some embodiments, the testing area on the porous membrane is enclosed into a specific area through the liquid blocking element, for example, the specific area is similar to a reservoir or a liquid storage pool; the liquid can be directly dripped from the porous membrane to the reservoir or the liquid storage pool, such that the liquid has a specific height on the surface of the porous membrane. Thus, the liquid in the reservoir or the liquid storage pool can cover all the testing units at the same time. In some embodiments, the liquid here is a liquid including the reaction reagent reacting with the label. When the reaction reagent needs to be allowed to contact with the label for a long time, the label is allowed to be fully reacted and consumed, thereby obtaining the accurate results. The time herein is somewhat longer than the time when the reaction liquid enters from one end of the porous membrane and flows out from the downstream of the testing area. In some embodiments, when the liquid is located in the liquid storage pool enclosed by the liquid blocking element, the water absorption area still maintains the ability to absorb water to the porous membrane, and the liquid in the liquid storage pool will also flow into the water absorption area due to the capillary action of the water absorption area. It can be understood that the reaction liquid contacts with the plurality of testing units on the testing area to react, and continuously flows through the porous membrane to the water absorption area. When the reaction liquid is completely absorbed by the water absorption area, basic reaction is completely achieved. The reaction time of the reaction liquid can depend on an amount of the reaction liquid.

In some embodiments, the label is the enzyme; the second receptor is labeled by the enzyme; when the second enzyme-labeled receptor and the first receptor bind to the analyte to form the complex for specific binding, the labeled enzyme will also be brought into the immune complex and immobilized onto the testing unit. After a reaction area ends up reaction, the second enzyme-labeled receptor remaining in the reaction area can be cleaned by the washing liquid; subsequently, the reaction liquid corresponding to the labeled enzyme is added and can be catalyzed by the enzyme to generate visible substances, fluorescent substances or shimmering substances in visible light if contacting with the enzyme-labeled immune complex, and these substances can be observed by naked eyes or by measuring fluorescence and shimmering signals with sensors. In some embodiments, the reaction liquid can be directly dropped into the reservoir formed by the liquid blocking element for reaction. In some embodiments, the liquid blocking element is coated with a thin hydrophobic polymer plastic sheet, and conforming materials include but are not limited to polyvinyl chloride (PVC), polyester (PET) and polystyrene (PS) sheets. The liquid blocking element is adhered to the surface of the porous membrane by an adhesive. In some embodiments, the liquid blocking element itself has some elasticity, and the elasticity is adjusted by the materials and the adhesive to ensure close adhesion of the liquid blocking element and a shell and eliminate the risk of liquid side leakage. In some embodiments, the function of the liquid blocking element is achieved by the window areas of the upper strip and the lower strip of the test strip and is not provided separately.

In some embodiments, if the enzyme is regarded as a sphere, the size of the labeled enzyme is related to the molecular weight of the enzyme itself. For example, horseradish peroxidase (HRP) has a molecular weight of approximately 40kD and a molecular diameter of approximately 4nm; and recombinant alkaline phosphatase (AP) has a molecular weight of approximately 80kD and a molecular diameter of approximately 5nm. If the immune complex exists in a form of membrane-antigen-antibody (analyte)-antibody (analyte antibody)-enzyme, the thickness of the antibody-antibody-enzyme relative to the membrane-antigen in the immune complex is only increased by approximately 16nm and much smaller 8µm (8,000nm) than the pore size of the porous channel on the membrane, and the enzyme-labeled immune complex generated will not block the internal channel of the membrane or prevent the test liquid reagent from continuing to diffuse backwards and make immune reaction with a posterior binding site.

For the membrane with the pore size of 8µm as mentioned above, the pore size is measured by an electron microscope. In practical use, the membrane size can be judged according to the lateral flow rate of the liquid in the membrane. The pore size of the membrane is inversely proportional to the lateral diffusion time of the liquid in the membrane per unit distance. The liquid laterally diffuses by 4cm in the 8µm membrane for approximately 140 seconds; the short consumed time of the lateral diffusion indicates the pore size of the membrane being greater than 8µm, otherwise, the pore size of the membrane being smaller than 8µm.

In some embodiments, the present invention provides an immune reaction for lateral diffusion test and a using method thereof. Thus, this can avoid some problems in the prior art, for example, samples need to be added with respect to a plurality of testing indicators many times. However, in the chip and the using method thereof provided by the present invention, the samples need to be added only once, such that the present invention is more convenient for operation and applicable to a case where the samples are scarce; and it is more intuitive to reflect the progress of immune reaction through enzyme labeling.

The above method cannot be used in the existing chip. The main reasons lie in that: a plurality of bonding sites will interact with each other, and the anterior testing area have immune reaction, and the influence of the posterior testing area cannot be ignored, resulting in determination problems. Even in extreme cases, the plurality of bonding sites do not interact with each other and liquid may flow away from the surface, such that only a small amount of the liquid does not react with the bonding sites, seriously affecting the determination accuracy. However, the immune reaction chip for lateral diffusion test and the using method thereof provided by the present invention can solve these problems, and the principle has been explained above.

In some embodiments, the pore size of the porous membrane is more than 8µm. In some embodiments, the membrane material includes, but is not limited to, nitrocellulose membrane, polyvinylidene fluoride membrane, modified nylon membrane, or polyphenylene polyethersulfone membrane.

In some embodiments, the water absorption area includes materials with water absorption function, including but not limited to cellulose filter paper and cellulose acetate block. In some embodiments, that dilution capacity of the water absorption area is greater than or far great than the water absorption capacity of the porous membrane. In some embodiments, an amount of the liquid that can stay in the liquid absorption material is much larger than the sum of the liquid flowing into the liquid sample contact area of the membrane and the reaction liquid flowing into the reaction area or the sum of the liquid discharged from the liquid discharge area of the membrane.

In some embodiments, the device further includes a compartment assembled by an upper strip and a lower strip, thereby protecting the absorbent material and part of the porous membrane and only exposing the testing area; the testing area exposed through the window of the upper strip is used for reading the testing results. In some embodiments, the first receptor or the second receptor can include, but is not limited to, polypeptides, proteins, glycoproteins, lipoproteins, nucleic acids and protein complexes that bind to the nucleic acids, as well as antibodies, antigens, or any other substances that can be used to directly or indirectly test the analytes in the liquid sample through specific binding.

In another aspect of the present invention, a method for testing an analyte in a liquid sample is provided, and the method includes: providing a testing device, where the testing device includes a porous membrane and a water absorption area in fluid communication with the porous membrane; the porous membrane has a first end and a second end; the second end is connected with the water absorption area; the testing area is provided at the first end of the porous membrane and the second end thereof; the testing area includes one or more first receptors for testing the presence or absence or quantity of one or more analytes in the liquid sample thereon; one end of the porous membrane is allowed to contact with the liquid sample; the liquid sample is allowed to flow on the porous membrane and flow through the testing area, binds to the first receptor on the testing area to form a complex, and then flows into the water absorption area.

In some embodiments, after the first end of the porous membrane contacts with the liquid sample, the first end of the porous membrane is allowed to contact with the second receptor with a label, such that the second receptor flows along the porous membrane and flows through the testing area under the action of the water absorption area, and binds to the complex in the testing area to be immobilized on the testing area.

In some embodiments, the reaction liquid includes substances that can be catalyzed by enzymes to generate chemiluminescence, fluorescence precipitation or chromophore precipitation visible to the naked eye under visible light. In some embodiments, a technical method for testing an analyte in a liquid sample in the testing area of the present invention includes an immune reaction mode, including but not limited to a one-step sandwich method, a two-step sandwich method, an indirect method, and a blocking competition method. In some embodiments, the testing area can also include a technical method suitable for hybridization test, including but not limited to a DNA mixture with a same sequence or complementary sequence amplified by PCR for determining DNA, and a DNA mixture with a same sequence or a complementary sequence amplified by reverse PCR for determining RNA. In some embodiments, the immune reaction chip element reacts with different liquids or liquid reagents to form different intermediates, such as protein-protein complexes, protein-protein-labeled protein complexes and protein-labeled protein complexes. In some embodiments, intermediate complexes formed in nucleic acid hybridization experiments are DNA-DNA-FITC complex, DNA-DNA-Biotin complex, DNA-DNA-Bition-streptavidin-enzyme complex, and DNA-DNA-FITC-Anti-FITC antibody-enzyme.

In some embodiments, after the first end of the porous membrane is allowed to contact with the liquid sample, the porous membrane is allowed to contact with the washing liquid to remove impurities on the testing area or the liquid sample remaining on the testing area.

In some embodiments, the first end of the porous membrane is allowed to successively contact with the liquid sample, a first washing liquid, the solution including second receptor with a label, a second washing liquid, and the reaction liquid, where the reaction liquid includes a reaction reagent capable to react with the label to generate signals.

In some embodiments, after contacting with the liquid sample, or after contacting with the first washing liquid, the first end of the porous membrane is allowed to contact with the blocking liquid including the blocking reagent. In some embodiments, the blocking reagent includes protein, polypeptide or protein fragments.|

In some embodiments, the liquid blocking element is provided upstream of the testing area, covers the porous membrane, and is exposed of the first end of the porous membrane, thereby forming one end contacting with the liquid. In some embodiments, the liquid is allowed to enter from the upstream of the liquid blocking element, flows through the porous membrane and the testing area on the porous membrane, and then flows into the water absorption area. The liquid blocking element allows the liquid from the first end to flow through the porous membrane, instead of flowing above the surface of the porous membrane.

In some embodiments, a second liquid blocking element is provided between the water absorption area and the testing area, such that the liquid on the porous membrane stays on the porous membrane for more time, allowing the liquid to contact with the testing area for more time. Therefore, the second liquid blocking element is provided to reduce the flow rate of the liquid. The function of the second liquid blocking element can also be understood as slowing down the normal flow rate of the liquid. In some embodiments, the liquid blocking element applies a specified pressure to the porous membrane, thereby allowing the porous membrane to be compressed and slowing down the flow rate of the liquid on the porous membrane, without completely cutting connection between the porous membrane and the water absorption area. In some embodiments, the water absorption area may continue to absorb the liquid from the porous membrane.

In some embodiments, an area where a reservoir is located is enclosed on the porous membrane through the liquid blocking element; the testing area is located in such an area; and the reaction liquid is directly added into the reservoir and is allowed to contact with the entire testing area. In some embodiments, the reaction liquid includes the reaction reagent, and the reaction reagent can react with the label to generate the signal substance. Therefore, in some embodiments, after the solution including second receptor with a label flows through the testing area, the reaction liquid is directly added into the reservoir, and the reaction reagent in the reaction liquid reacts with the label immobilized on the testing area to generate the signal substance. If the label is the enzyme, the reaction reagent can react with the enzyme, and the signal substance generated is a colored precipitant.

In some embodiments, the reaction liquid is directly added to the reservoir, and allowed to cover the testing area to form a layer of the reaction liquid that stay here for a period of time. The time may be 10 seconds to 60 minutes. In some embodiments, when the reaction liquid is allowed to stay in the testing area, the water absorption area can continue to absorb the reaction liquid from the membrane until the water absorption area absorbs all the reaction liquid into the water absorption area.

In a third aspect of the present invention, a device for testing an anti-allergic antigen is provided, and the device includes a testing area and a water absorption area, where the testing area is used to directly contact with liquid, and the water absorption area is located downstream of the testing area and used to absorb the liquid from the testing area; the liquid is allowed to flow laterally through the testing area and then flow onto the water absorption area; and a plurality of first receptors specifically binding to an anaphylactic antibody in the liquid sample are immobilized onto the testing area, and the first receptors may be a first antibody of an allergen or anaphylactic antibody. The plurality of different first receptors bind to a plurality of different allergen antibodies in the liquid sample. In some embodiments, the testing area is located on the porous membrane. In some embodiments, one end of the porous membrane is used to directly contact with the liquid and the other end thereof is used to contact with the water absorption area, such that the water absorption area is allowed to absorb the liquid on the porous membrane, and the liquid is allowed to flow from one end of the porous membrane contacting with the liquid to the other end of the porous membrane contacting with the water absorption area through the testing area. In some embodiments, the liquid includes one or more of a diluent for dissolving or diluting the liquid sample, a liquid including a label, a washing liquid, and a blocking liquid. In some embodiments, one end of the porous membrane directly contacting with the liquid may continuously contact with one or more of the liquid sample, the liquid including a label, the washing liquid, the blocking liquid, and the reaction liquid capable to react with the label to generate a signal substance. In some embodiments, one end of the porous membrane may successively contact with the liquid sample, the first washing liquid, the blocking liquid, a label-including anti-allergic antibody liquid, the second washing liquid, and the reaction liquid capable to react with the label to generate the signal substance.

In some embodiments, the liquid including a label includes a plurality of different second receptors that include the label and can specifically bind to the allergen antibody (IgE), and the second receptors may be the antibody of the allergen antibody (IgE) and other complexes that can specifically bind to the first receptor and the allergen antibody. The antibody of the anaphylactic antibody can specifically bind different allergen antibodies in the sample. In some embodiments, each of the antibodies includes a same label. In some embodiments, the label includes enzyme, colored particles or dyes, or a fluorescent label, and preferably enzyme.

In some embodiments, the device further includes a liquid blocking element, where the liquid blocking element enables the reaction liquid to stay in the testing area on the porous membrane or enables the reaction liquid entering from one end of the porous membrane to flow through the porous membrane without flowing from the surface thereof as much as possible, and then flow through the testing area. In some embodiments, a first liquid blocking element is between one end of the porous membrane contacting with the liquid and the testing area, and allows the liquid entering from the end of the porous membrane to flow through the porous membrane without flowing from the surface thereof and then flows through the testing area. In some embodiments, the liquid blocking element is provided between the downstream of the testing area and the water absorption area, thereby slowing down the flow rate of the liquid and allowing the liquid to stay in the testing area. In some embodiments, an area where a reservoir is located is enclosed on the porous membrane through the liquid blocking element; the testing area is located in such an area; and the reaction liquid is directly added into the reservoir and is allowed to contact with the testing area. In some embodiments, the reaction liquid includes the reaction reagent, and the reaction reagent can react with the label to generate the signal substance. Therefore, in some embodiments, after the solution including second receptor with a label flows through the testing area, the reaction liquid is directly added into the reservoir, and the reaction reagent in the reaction liquid reacts with the label immobilized on the testing area to generate the signal substance. If the label is the enzyme, the reaction reagent can react with the enzyme, and the signal substance generated is a colored precipitant.

In some embodiments, the liquid blocking element is substantially composed of hydrophobic polymer materials, and the hydrophobic polymer materials comprise one or more of polyvinyl chloride, polyester, polyurethane, and polystyrene. In some embodiments, the liquid for dissolving a liquid sample does not include the blocking reagent. In some embodiments, the blocking reagent is provided to block a porous site of a blank area on the porous membrane. In some embodiments, the blocking reagent includes casein. In some embodiments, the porous membrane comprises a nitrocellulose membrane or a polyvinylidene fluoride membrane. In some embodiments, pure water is used as liquid to take less than 5 minutes to diffuse 4cm along the porous membrane. In some embodiments, the water absorption area includes an absorbent pad; the absorbent pad includes hydrophilic fiber materials; and the hydrophilic fiber materials are selected from natural plant fibers or chemically synthesized acetate fibers. In some embodiments, the liquid sample is a serum, plasma or whole blood sample.

In a fourth aspect of the present invention, a method for testing an allergen antibody in a liquid sample is provided, and the method includes: providing a testing device, where the device includes a testing area and a water absorption area; a plurality of first receptors are immobilized onto the testing area in an array, and each of the first receptors is capable to specifically bind to one allergen antibody in the liquid sample; and the testing area is located on the porous membrane; where the porous membrane is provided with a first end and a second end, the water absorption area is connected with the first end of the porous membrane, and the second end of the porous membrane is allowed to contact with the liquid sample, such that the liquid sample is allowed to flow through the testing area on the porous membrane and then flows into the water absorption area.

In some embodiments, the second end of the porous membrane is allowed to contact with a blocking liquid including a blocking reagent, and the blocking reagent is allowed to fill a blank area on the porous membrane or block a non-specific binding site. In some embodiments, the second end of the porous membrane is allowed to contact with a plurality of solutions including second receptor with a label, and each of the solutions including second receptor is capable to specifically bind to the allergen antibody in the liquid sample, such that the solutions including second receptor with a label flow into the water absorption area through the testing area under the capillary action of the water absorption area. In some embodiments, the porous membrane further includes an area formed by a liquid blocking element, the area includes the testing area therein, and the liquid blocking element enables a reaction liquid to stay in the testing area.

In some embodiments, the reaction liquid is directly applied to the testing area, such that the liquid blocking element enables the reaction liquid to stay in the testing area. In some embodiments, the reaction liquid includes a reaction reagent, and the reaction reagent is capable to react with the label to generate signals, and the presence or absence or quantity of analytes is obtained according to the presence or absence or quantity of the signals. In some embodiments, the signals are readable and preferably read by naked eyes. In some embodiments, when the reaction liquid stays on the testing area, the water absorption area is capable to absorb part of the reaction liquid on the porous membrane and the part of the reaction liquid flows onto the water absorption area. In some embodiments, the reaction liquid is allowed to stay in the testing area for 10 seconds to 60 minutes.

In some embodiments, after the second end of the porous membrane is allowed to contact with the liquid sample, the first end of the porous membrane is allowed to contact with a washing liquid and then a liquid including a label.

The present invention has the following beneficial effects:
1. The size of the enzyme molecule and the hydrophilic property of the surface do not affect the diffusion of the liquid in the membrane channel. Through the hydrophilic property, immune reaction for immobilization on a chip reaction area can be independently conducted, such that the one-dimensional motion of the liquid on the membrane can achieve the effect of the two-dimensional test, one sample can be tested once, and a large number of testing results of a plurality of indicators can be obtained.
2. According to the present invention, the concentration of an immune element during test is greatly increased due to the high carrying capacity of the immune element of the membrane, and the reaction time is shortened under the same sensitivity.
3. The liquid blocking element of the present invention can keep the reaction liquid and cover various binding sites in the chip, such that the enzyme-including immune complex can initiate catalytic reaction to simultaneously test the plurality of indicators.
4. According to the present invention, a sufficient amount of the liquid absorption material is used to provide driving force, such that the liquid automatically flows through the reaction area of the chip to complete the immune reaction and the necessary cleaning and separation; the process is a spontaneous process and does not involve in complicated manual operation and the assistance of the liquid path system of automatic equipment.
5. The present invention can be applied to the simultaneous determination of various substances in the fields of medicine, veterinary medicine, agriculture, animal husbandry, food safety, environmental monitoring, biological safety and the like, thereby obviously improving the working efficiency in these fields.

### BRIEF DESCRIPTION OF THE DRAWINGS

FIG. 1A is a schematic diagram showing a three-dimensional structure of a testing device according to a specific embodiment of the present invention.
FIG. 1B is a schematic diagram showing a three-dimensional structure of a testing device according to a specific embodiment of the present invention, where liquid is dripped into a liquid sample contact area 202.
FIG. 1C is a schematic diagram showing a three-dimensional structure of a combination of a porous membrane and a liquid blocking element of a testing device according to a specific embodiment of the present invention.
FIG. 2A is a schematic diagram showing a three-dimensional structure of a testing device according to another specific embodiment of the present invention.
FIG. 2B is a schematic diagram showing a three-dimensional structure of a combination of a porous membrane and a liquid blocking element according to a specific embodiment of the present invention.
FIG. 3 is an exploded diagram showing a three-dimensional structure according to a specific embodiment of the present invention.
FIG. 4A is a schematic diagram showing a three-dimensional structure of an operation of a testing device according to a specific embodiment of the present invention.
FIG. 4B is a schematic diagram showing addition of a reaction liquid to a testing area according to a specific embodiment of the present invention.
FIG. 4C is a schematic diagram showing a case that color appearing in a testing area indicates a testing result when a reaction liquid is added to a testing area according to a specific embodiment of the present invention.
FIG. 5A shows a picture example in which a plurality of testing units are distributed in a testing area in an array according to a specific embodiment of the present invention.
FIG. 5B shows a testing result picture example in which a plurality of testing units are distributed in a testing area in an array according to a specific embodiment of the present invention.
FIG. 6 is an exploded diagram showing a three-dimensional structure of a testing device according to a specific embodiment of the present invention.
FIG. 7A - FIG. 7D show pictures of testing results under different sample dilution ratios according to a specific embodiment of the present invention.
FIG. 8A - FIG. 8C show pictures of testing results of generation of a chemiluminescent signal according to a specific embodiment of the present invention.
FIG. 9 is a diagram showing a testing result that a reaction liquid is allowed to contact with one end of a porous membrane receiving liquid and then flow through the porous membrane according to a specific embodiment of the present invention (method 1, a two-sided immune reaction chip element is adopted, where a two-sided type means that two liquid blocking elements are arranged on the porous membrane, with a specific structure as shown in FIG. 3).
FIG. 10 shows a diagram of a result that a reaction liquid is directly dripped onto a testing area for reaction test according to a specific embodiment of the present invention (method 2, a two-sided immune reaction chip element is adopted, where a two-sided type means that two liquid blocking elements are arranged on a porous membrane, with a specific structure as shown in FIG. 3).
FIG. 11 shows a result picture in which different liquids are used for reaction (results after 25 minutes) when a reaction liquid contacts with a chip reaction area (a two-sided immune reaction chip element is adopted, as shown in FIG. 3) according to a specific embodiment of the present invention.
FIG. 12 is a schematic diagram showing a final result of contact between a reaction liquid and a chip reaction area according to a specific embodiment of the present invention (a two-sided immune reaction chip element is adopted, as shown in FIG. 3) (results after 10 minutes).
FIG. 13 is a structural diagram of an assembled testing device for testing an allergen according to another specific embodiment of the present invention (an allergen test).
FIG. 14 shows a solution tank composed of a plurality of chambers for accommodating liquids according to a specific embodiment of the present invention, where the liquids include a sample diluent, a blocking liquid, a washing liquid, and a liquid including a label.

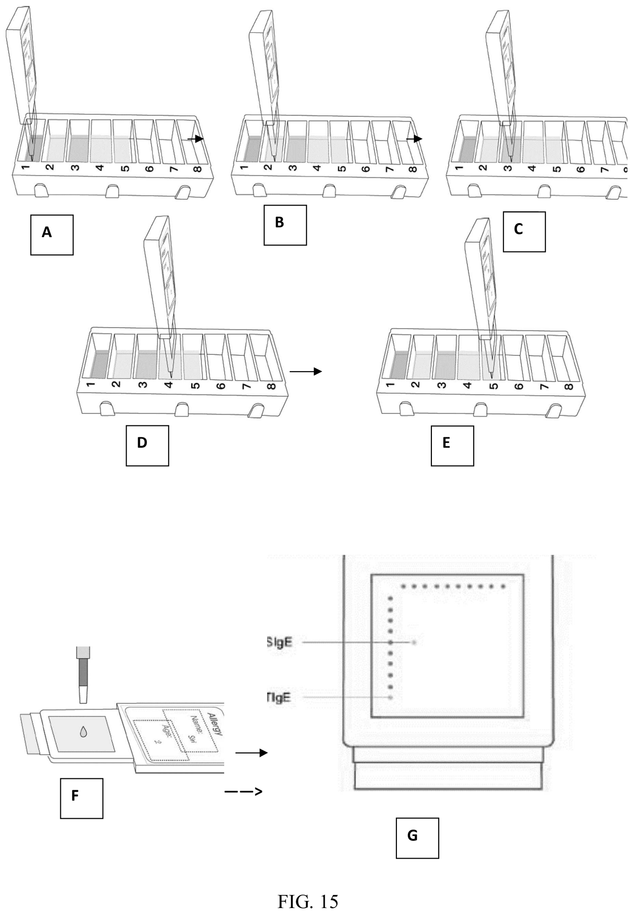

FIG. 15 is a schematic diagram showing specific operation steps (as described in Embodiment 5), where Step A is to insert a washing liquid end of a test strip into a No. 1 solution tank 10 to contact with a sample solution therein, Step B is to insert the washing liquid end into a No. 2 solution tank 20, Step C is to insert the washing liquid end into a No. 3 solution tank 30, Step D is to insert the washing liquid end into a No. 4 solution tank 40, Step E is to insert the washing liquid end into a No. 5 solution tank 50, Step F is to directly drip a reaction liquid into a testing area, and Step G is to read results on the testing area and determine a total amount of IgE antibodies or the presence or absence thereof.

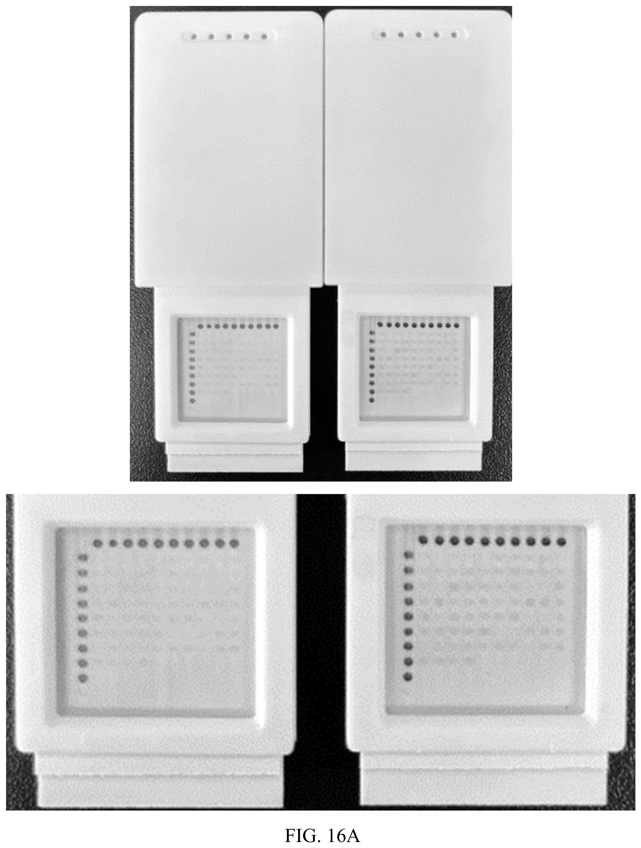

FIG. 16A is a test effect diagram (false positive) showing a test sample application end not contacting with a blocking liquid, where an original picture is at the top and an enlarged picture of corresponding results is at the bottom, it can be clearly seen that the false positive appears in a testing unit with a colored background, indicating that a blocking reagent may be necessary for allergen test.

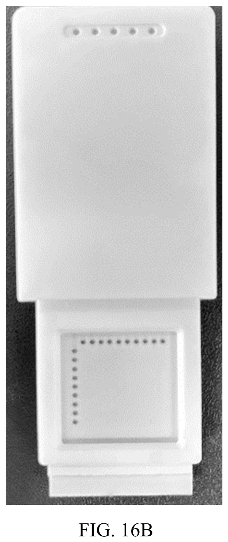

FIG. 16B is a test effect diagram showing a test sample application end contacting with a blocking liquid, with a clean background and correct negative display.

### DETAILED DESCRIPTION OF THE INVENTION

The structures involved in the present invention or the technical terms used are further explained below. Unless otherwise specified, they shall be understood and explained according to the general terms commonly used in the art. The following further elaborates preferred embodiments of the present invention with reference to the accompanying drawings, and it should be noted that the embodiments described hereinafter are intended to facilitate the understanding of the present invention and do not impose any restriction on it. All features disclosed in the embodiments of the present invention or all steps of methods or processes disclosed therein can be combined in any way except mutually exclusive features and/or steps.

### Detection

Detection means to assay or detect presence or absence of a substance or material, including but not limited to, a chemical substance, an organic compound, an inorganic compound, a metabolite, a drug, a drug metabolite, an organic tissue, a metabolite of an organic tissue, a nucleic acid, a protein or a polymer. In addition, detection means that the amount of a substance or material is tested. Further, assay also means immunoassay, chemical assay, enzyme assay, and the like.

### Samples

Specimens tested or collected by the test device of the present invention include biological liquids (for example, case liquids or clinical specimens), such as liquid samples or solid samples. Liquid specimens or liquid samples, or fluid samples or fluid specimens may be derived from solid or semi-solid specimens, including feces, biological tissues and food specimens. The solid or semi-solid specimens may be converted to liquid specimens by any appropriate methods, such as mixing, mashing, macerating, incubating, dissolving, or digesting the solid specimens by enzymolysis in suitable solutions, such as water, phosphate solutions, or other buffer solutions. "Biological samples" include animal, plant, and food derived samples, including, for example, human or animal derived urine, saliva, blood (whole blood, plasma, serum, and the like) and components thereof, spinal fluid, vaginal secretions, sperm, feces, sweat, secretions, tissues, organs, tumors, cultures of tissues and organs, cell cultures, and media. Preferably, the biological specimen is urine; and preferably, the biological specimen is saliva. Food specimens include food processed materials, final products, meat, cheese, wine, milk, and drinking water. Plant specimens include specimens derived from any plants, plant tissues, plant cell cultures, and media. "Environmental specimens" include specimens derived from the environment (for example, liquid specimens from lakes or other bodies of water, sewage specimens, soil specimens, groundwater, seawater, and waste liquid specimens). The environmental specimens may further include sewage or other waste water. The solid samples here include specimens including no water or little water, such as solid soil, animal and plant tissues, or drug powder, such as drug of abuse powder samples.

An appropriate test device according to the present invention can be used to test any analyte. Preferably, the test device of the present invention is used to test drug small molecules in saliva or urine, or viruses in powder specimens or blood samples, such as human immunodeficiency virus (HIV) and coronaviruses. The sample here can be in any form or type of sample. If the sample is solid, it can be treated with a liquid for dissolving a liquid sample; alternatively, if the sample existing in a form of solution is liquid, it can be directly tested; of course, the liquid sample can be diluted, dissolved and treated to form a solution including the liquid sample, so as to test or assay the device of the present invention. Therefore, the liquid sample here includes a sample that is itself a liquid, or a liquid sample formed by dissolving a solid with a solution, a sample (liquid or solid) that has been diluted with a diluent to form a liquid sample, and a sample that has been pretreated to form a liquid sample. In some embodiments, the sample is added into a dilute solution, and then one end of the porous membrane contacting the sample also falls within a specific embodiment of the present invention.

### Downstream and upstream

Downstream or upstream is divided according to a flow direction of a liquid. Generally, a liquid flows to a downstream area from an upstream area. The downstream area receives the liquid from the upstream area, and a liquid also may flow to the downstream area along the upstream area. Here, downstream or upstream is generally divided according to a flow direction of a liquid, for example, on some materials where capillary force is utilized to facilitate the flow of a liquid, a liquid may overcome gravity to flow towards an opposite direction to the gravity; and in this case, downstream or upstream is divided according to a flow direction of the liquid. For example, in the testing device of the present invention, when one end of the porous membrane contacts with the liquid, the liquid flows from one end of the porous membrane to the other end thereof by a capillary force and flows through the testing area on the porous membrane; if the liquid here is a liquid sample, a first receptor immobilized on the testing area specifically binds to an analyte (if any) in the liquid sample.

In some embodiments, after the liquid sample flows through the porous membrane and the porous membrane is moistened, if it is desired that one end of the porous membrane contacts with another liquid, for example, a washing liquid, a blocking liquid, or other liquids for testing, in this case, the capillary force needs to be provided through a water absorption area communicated with the porous membrane, such that the liquid can continue to flow on the porous membrane. Thus, flowing depends on the water absorption area, and different kinds of liquids can be allowed to flow on the porous membrane many times from one end of the porous membrane to the other end thereof through the testing area. "many times" here can mean that a same solution flows many times, or that different solutions are absorbed by the porous membrane and flow, which will be described in detail below.

### Gas communication or liquid communication

Gas communication or liquid communication means that liquid or gas can flow from one place to another. In the flow process, the liquid or gas may pass through some physical structures that play a guiding role. The "pass through some physical structures" here means that the liquid passes through the surface of these physical structures or the internal space of these physical structures and flows to another place passively or actively, where the passive flow is usually caused by external forces, such as the flow under the capillary action. The flow here may also be a flow due to self-action (gravity or pressure) of the liquid or gas, and also may be a passive flow. The communication here does not mean that a liquid or a gas is necessarily present, but indicates a relationship or state between two objects under some circumstances. If a liquid is present, it can flow from one object to another. Here it means the state in which two objects are connected. In contrast, if there is no liquid or gas communication state between two objects, and the liquid is unable to flow into or onto another object, it is a non-communication, non-liquid communication or non-gas communication state.

### Analyte

Examples that can use an analyte related to the invention include some small-molecule substances, including drugs (such as drug of abuse). "Drug of Abuse" (DOA) refers to the use of a drug (typically functions to paralyze the nerves) not directed to a medical purpose. Abuse of these drugs will lead to physical and mental damage, dependency, addiction and/or death. Examples of abuse of drug include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium^{®}, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The test device of the invention may also be used for detecting drugs belonging to a medical use but easy to be taken excessively, such as tricyclic antidepressants (imipramine or analogues) and acetaminophen. These drugs are metabolized into micromolecular substances after absorbed by human body. These micromolecular substances exist in blood, urine, saliva, sweat and other body fluids or in some body fluids.

For example, the analyte detected by the present invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus*, *Salmonella*, *Fusiformis*, *Camyplobacter genus, L. monocytogenes*, *Vibrio*, or *Bacillus cereus*) and substances related with physiological features in a urine sample, such as pH and specific gravity. Chemical analysis of any other clinical urine may be performed by lateral flow detection in combination with the device of the present invention.

In some embodiments, the device of the present invention can be used to once test at least two or more analytes, for example, 5, 10, 15, 20, 30, 40, 50, 60 and 100 different analytes can be tested once. When a plurality of analytes are tested with a high-throughput detection technology, a plurality of testing units can be arranged on the testing area on the porous membrane; these testing units are arranged in an array and each testing unit can test one analyte. If there are 20-50 testing units, 20-50 analytes can be tested once. In some embodiments, the analyte includes allergens to be tested, which may be 20-100 allergens if possible. The following makes detailed explanations.

The analyte here can also be in the sense of nucleic acid, such as a nucleic acid fragment in a sample, or an amplified nucleic acid fragment, such as DNA, mRNA, or any other nucleic acid fragment, and can also be tested by the device of the present invention. Of course, it can also be an analyte in the sense of protein or polypeptide. For example, the device of the present invention can be used to once test more than 20-200 different proteins or different polypeptide fragments in a sample, and of course, it can also be different immune proteins in the sample. Of course, it can also be a substance for testing small chemical molecules in a sample, for example, 20-200 different small molecular compounds can be tested once. In some embodiments, the analyte of the present invention also includes the analyte after the sample is processed, for example, the nucleic acid fragment is amplified, or the sample is pretreated by filtration, or the liquid sample that may include the analyte after the sample is diluted.

### Flow of liquid

Typically, the flow of liquid refers to flow from one place to another. Under normal circumstances, the flow of liquid in nature mostly is from a high place to a low place under the action of gravity. Flow herein also depends on external force (namely, external gravity), and thus it can become flow under normal gravity. In addition to gravity, the flow of liquid may also overcome gravity to move from a low place to a high place. For example, the liquid can flow from a low place to a high place through extraction, compression or pressure received by it. Alternatively, the liquid may flow against its own gravity in relation to pressure. Of course, the liquid can flow from a low place to a high place under the capillary force.

### Porous membrane and water absorption area

In some embodiments, the porous membrane of the present invention has water absorption, that is, the porous membrane can absorb water under its capillary action; liquid can be allowed to flow on the porous membrane, and any material that can allow the liquid to flow under the capillary action can be used as the porous membrane of the present invention. The term "on" here mainly means that the liquid flows through the porous membrane, and also means that the liquid flows on the surface of the porous membrane. In some embodiments, the term "on" here means that the liquid is allowed to flow through the porous membrane to minimize the liquid flowing on the surface of the porous membrane. Because the porous membrane has a thickness, the liquid usually moistens the whole membrane or flows through the membrane with a thickness. When a testing area is arranged on the membrane, first receptors are immobilized on the testing area. These receptors are generally not located on the surface of the porous membrane but in the pores thereof, and the sites that specifically bind to the analyte on the receptors are exposed. If the liquid only flows from the surface, the binding time of the receptor and the analyte is shortened due to the fast flow rate of the liquid, such that the receptor is incompletely bound to the analyte, resulting in false negative results. However, if the liquid flows through the porous membrane, the porous membrane can be completely moistened by the liquid and the flow rate of the liquid naturally slows down, increasing contact time between the liquid and the first receptor, allowing the analyte to be bond to the site more fully, thereby increasing test accuracy.

The "water absorption" used here means that these materials can absorb a water-based solution and allows the liquid to flow on or in the porous membrane mainly depending on the capillary force. The term "water" here can be any solution including H₂O molecules, for example, purified water, water including trace elements, or sterile water. Of course, the water also includes liquid samples, biological samples, or solutions for treating solid samples with water as a solvent. The solution or liquid here also includes one or more of a liquid sample, an eluate, a blocking liquid, a reaction liquid, or a washing liquid, as well as any other solution tested, such as a solution including second receptor with a label.

The term "porous" here is a broad meaning and can mean that some materials have multiple micropores or can be similar to a filter paper; the filter paper has micropores or capillary fibers thereon, or micropores formed on some materials through any process (such as engraving, printing, and other laser technologies), but these micropores are distributed on a substrate; the substrate has water absorption and can allow the liquid to flow on the substrate under the capillary action. These can be regarded as a specific embodiment of the porous membrane defined by the present invention.

In some embodiments, the porous membrane is allowed to contact with the liquid in the beginning, for example, the liquid sample; the liquid can be allowed to flow on the porous membrane under the capillary action of the porous membrane, such that the porous membrane is moistened or completely moistened. The "contact" of the present invention means that the porous membrane is allowed to contact with the liquid, such that the liquid flows on the porous membrane or covers the porous membrane. A contact mode can be that the liquid is allowed to dripped onto the porous membrane or one end of the porous membrane is immersed in the liquid, or the liquid can be directly dripped onto the testing area on the porous membrane, and a liquid level with a specified height is formed on the testing area.

When it is desired that the porous membrane moistened by the liquid continues to contact with the liquid, the liquid can continue to flow on the porous membrane and flow through the testing area, which provides necessary reagents or conditions for assaying the analyte in the testing area; in this case, the moistened membrane substantially has no capillary force or substantially loses capillary force. Therefore, it is necessary to provide the driving force for liquid flow with another measure having water absorption capacity. Therefore, In some embodiments, a water absorption area is provided and communicated with the porous membrane, such that the liquid can continue to move or flow on the moistened porous membrane through the capillary force of the water absorption area. In some embodiments, the water absorption area has the absorbent material; the absorbent material has the much greater liquid absorption capacity than the porous membrane; alternatively, the saturated absorption volume of the absorbent material in the water absorption area is greater than or far greater than that of the porous membrane. Thus, the liquid still can be allowed to flow through the porous membrane even if the moistened porous membrane loses the capillary force. This is due to a fact that the water absorption area has great or much capillary force or great saturated liquid absorption capacity. The "saturated" here means the maximum water absorption volume of materials with capillary water absorption capacity that can continue to absorb water without the capillary force, or the maximum water absorption capacity or maximum water storage capacity of materials with capillary water absorption capacity. For example, the volume of the porous membrane that is moistened by the liquid and loses the capillary force is 1mL; the volume of the absorbent material that is located in the water absorption area and loses the capillary force or the saturated liquid absorption volume thereof is 2mL, 10mL, 20mL, 50mL, 100mL, 200mL, or 500mL; thus, when 1mL liquid is applied to the porous membrane, the porous membrane can be moistened by the 1mL liquid through the capillary force thereof; when it is desired that the moistened porous membrane continues to absorb the liquid, the liquid is allowed to flow on the porous membrane, and it is necessary to provide the driving force through the absorbent material in the water absorption area, such that the liquid is allowed to continue to flow on the porous membrane through the driving force. It can also be understood that if it is desired that 10mL liquid flows through the porous membrane many times, the saturated absorption capacity of the absorbent material in the water absorption area is 10mL or greater than 10mL, for example 12mL-50mL. The "saturated absorption liquid" used here means a total amount of liquid absorbed by a material until such a material loses its capacity to absorb liquid at the maximum liquid absorption capacity through the capillary force. It can be understood that because the saturated absorption capacity of the absorbent material in the water absorption area is greater than or far greater than the water absorption capacity of the porous membrane, there is a difference in absorption or capillary force when the water absorption area is connected with the porous membrane. Thus, even if the moistened porous membrane loses its capacity to continue to absorb water, the porous membrane still can be allowed to continue to absorb more liquid through the capillary force of the absorbent material connected with the porous membrane, thereby allowing the liquid to flow through the moistened porous membrane. In some embodiments, relative to the absorbent material in the water absorption area, the porous membrane is generally a membrane with small thickness and uniformly distributed micropores; the absorbent material may be filter paper and fiber with great thickness and large saturated absorption liquid volume, thus realizing the function of the present invention. Thus, in some embodiments, the absorbent material has the greater thickness and volume than the porous membrane.

In some embodiments, in addition to being able to absorb the liquid or allowing the liquid to flow through the porous membrane, the porous membrane needs to be provided with the testing area thereon, and thus the testing area is a place for testing the presence or absence or quantity of the analytes in the liquid sample. In order to complete the function of the testing area, some substances need to be treated thereon. In some embodiments, first receptors are arranged on the testing area, and these first receptors are immobilized on the porous membrane instead of flowing with the liquid. In some embodiments, the testing area includes a plurality of testing units thereon; one first receptor is immobilized onto each testing area, for example, the first receptor immobilized onto each testing area can specifically bind to one analyte in the liquid sample directly or indirectly to form a complex; thus, a plurality of analytes can be once tested, and the sample is a same sample. In some embodiments, the testing area includes 10-100 testing units thereon; each testing unit corresponds to one analyte in the liquid sample; one first receptor that can specifically bind to one analyte in the liquid sample is immobilized onto each testing unit, and 10-100 different first receptors are immobilized onto 10-100 testing units, such that 10-100 different analytes in the liquid sample can be tested and a plurality of testing units are arranged on the testing area in an array-like form.

In some embodiments, the porous membrane may be a nitrocellulose membrane or a nylon membrane, or capillary micro-channels are formed on non-absorbent materials. In some embodiments, the porous membrane includes nitrocellulose membrane, polyvinylidene fluoride membrane, modified nylon membrane, or polyphenylene polyethersulfone membrane. The capillary channels formed by a process on non-absorbent materials here can also be immobilized onto the surface thereof to specifically bind (directly or indirectly bind) the analyte in the liquid sample, and this is also a specific embodiment of the porous membrane. Although the non-absorbent materials such as plastics, glass and ceramics do not absorb water, they have capillaries thereon, the liquid can flow thereon many times through the capillary force of the absorbent materials.

In some embodiments, when the testing area is located on the porous membrane, a testing result control area can also be located on the porous membrane. The testing result control area is mainly the area where the device or the result is tested to be effective. The testing result control area may also include a plurality of test control units that can be arranged around the testing area. In some embodiments, if the testing units on the testing area are distributed in the testing area in an array, the testing result control area can also be distributed around the testing area in an array, for example, distributed downstream of the testing area, or distributed to the left, right or upstream of the testing area.

In some embodiments, the testing area and the testing result control area are distributed on the porous membrane; one end of the porous membrane can be used to contact with the liquid, and the other end thereof is connected with the water absorption area, for example, the absorbent paper of the water absorption area is overlapped on the other end of the porous membrane. Thus, both the testing area and the testing result control area can be distributed on one porous membrane made of a same material; in addition, the porous membrane is used as a liquid contact area at the upstream of the testing area, and connected with the water absorption area at the downstream of the testing area. This way makes a production process simple. Of course, in some embodiments, the porous membrane can be a plurality of membranes made of different materials or with different pore sizes, for example, the testing area is distributed on a first membrane; a second membrane is distributed downstream of the testing area of the membrane and provided with the testing result control area; a third membrane is arranged upstream of the testing area and used as the liquid contact area; and the first membrane, the second membrane and the third membrane are superimposed on each other. This way is complicated, but it is still a feasible way. In addition to the above way, one end of the porous membrane for contacting with the liquid can also be connected with other porous diversion elements also made of porous materials, where the porous materials may be filter paper, glass fiber, and the like for guiding the liquid to flow to the porous membrane.

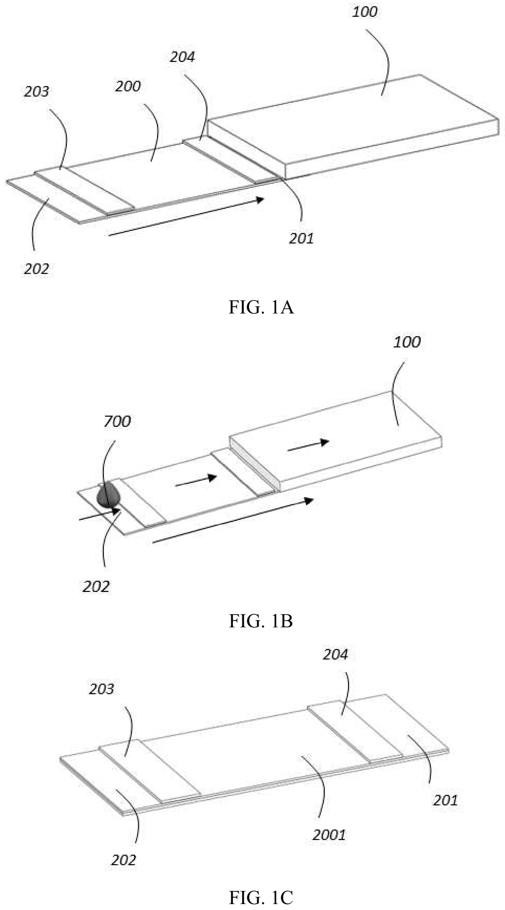

In some embodiments, the testing device as shown in FIG. 1A - FIG.3B includes the porous membrane 200 and the water absorption area in fluid communication with the porous membrane, and the porous membrane 200 can be arranged on a rigid liner or a bottom plate, and the testing area 2001 is arranged on the porous membrane. Here, the porous membrane is made of the same material, and is an integral membrane with divided areas to accomplish different functions. The testing area is provided at the center of the porous membrane, one end of the porous membrane is used to contact with the liquid sample contact area 202, the other end 201 thereof contacts with the water absorption area, and the testing area 2001 is provided at the center thereof. The liquid sample contact area 202 is provided upstream of the testing area, used to contact with the liquid, and can also be called a sample application area; this area is used to contact with different kinds of liquids, for example, a liquid sample, a washing liquid, a blocking liquid, a solution including second receptor with a label, or one or more washing liquids, or a reaction liquid that reacts with the label to generate signals. There is a specified time interval between these contacts of the liquids, for example, 1-5 minutes. Of course, there may be a time interval of 1-5 minutes between contact with one liquid and contact with another liquid. After the porous membrane 200 contacts with the liquid, the liquid can be allowed to flow on the porous membrane through the capillary force thereof, flow through the testing area 2001, and then flow to the downstream water absorption area 100. In this case, the liquid flows depending partly on the capillary force of the porous membrane and the capillary force of the water absorption area. After the porous membrane 200 is moistened by the liquid, if a liquid sample contact area 202 needs to continue to contact with another liquid, it is desired that the another liquid will continue to flow on the porous membrane. In this case, mainly through the capillary force of the water absorption area, the liquid is allowed to flow from the liquid sample contact area 202 to the testing area, flows to the downstream 201 of the testing area and then flow onto the water absorption area 100. The other end 201 of the porous membrane is contacted or covered or superimposed by the absorbent material in the water absorption area. In a flowing process, the liquid flows through the testing area 2001 and contacts with the testing unit in the testing area to generate binding reaction or chemical reaction, thereby testing the presence or absence or specific quantity of the analytes in the liquid sample.

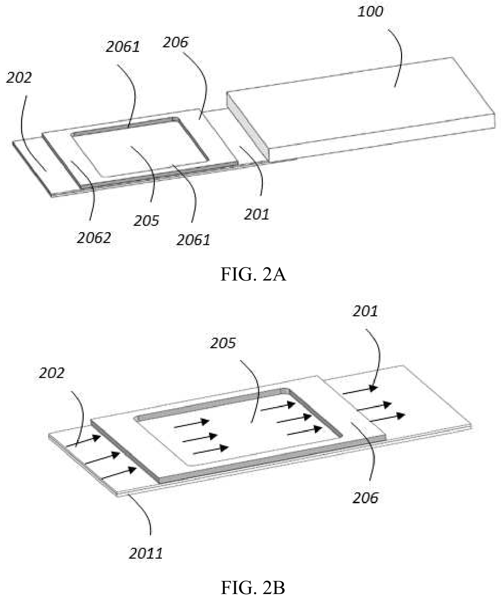

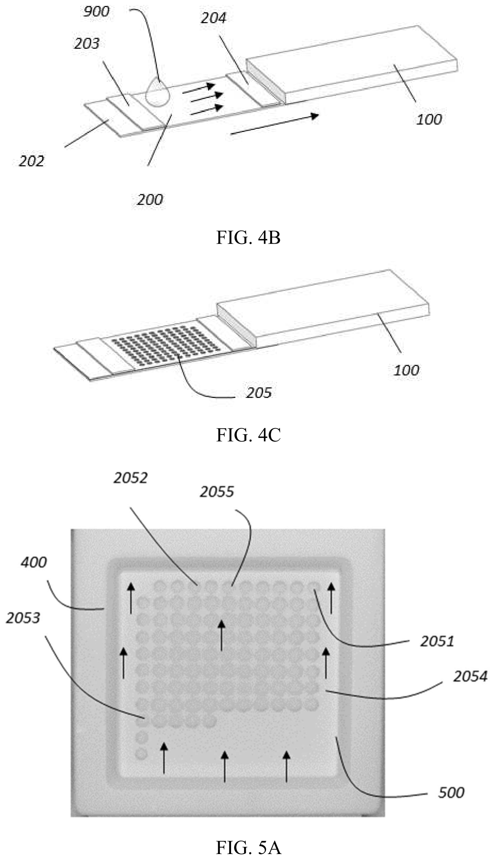

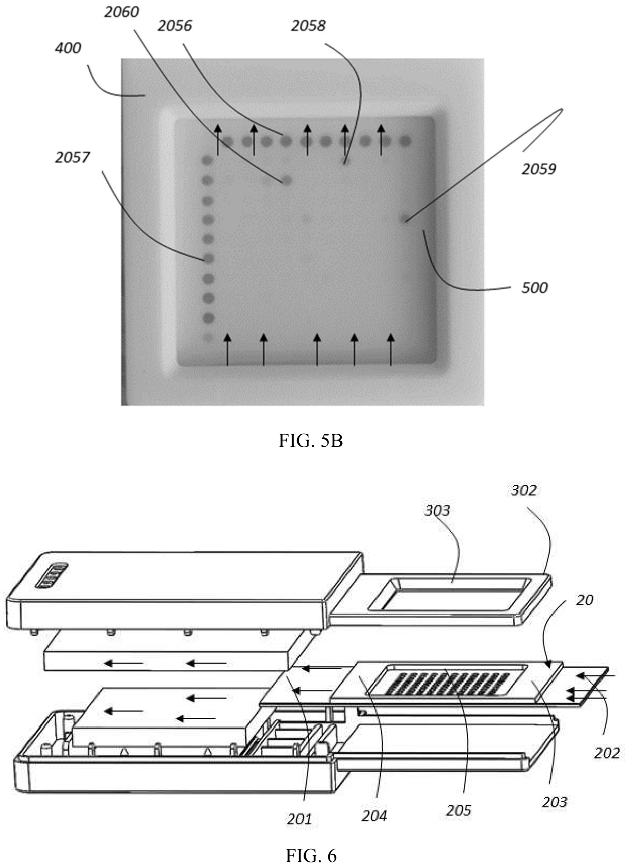

In some embodiments, a first liquid blocking element 203 is arranged on the porous membrane, covers the porous membrane and divides the porous membrane into one liquid sample contact area 202; and the liquid contact area is an area for contacting liquid 700 (as shown in FIG. 1B). As shown in FIG. 2A, the porous membrane includes an enclosed liquid blocking element thereon, and a similar liquid storage pool 2061 is formed on the porous membrane, so the liquid blocking element 2062 near one end of the porous membrane forms one liquid sample contact area 202 on the porous membrane. As shown in FIG. 4C, FIG. 5A and FIG. 5B, the porous membrane includes a plurality of testing units thereon, and each testing unit is used to test one analyte. As shown in FIG. 5A, the testing area 2001 has 9 rows of testing units, each row of testing units has 10-11 individual testing units 2052, 2055, and there is a specified distance between each testing unit. These places without the testing units can be called interval areas or blank areas 2052, or there is a blank area between each testing unit. The liquid flows from the liquid sample contact area 202 to the downstream thereof in an arrow direction, flows through the testing area, flows to the downstream 201 of the testing area, and is absorbed by the downstream water absorption area 100. These blank areas or interval areas, or testing units are mainly blocked by blocking reagents in subsequent operations to avoid false positive. This will be described in detail later. Specifically, the liquid enters from the lower end of FIG. 6A, and flows through a first row of testing units 2053, a second row of testing units, and a last row of testing units 2051 in sequence.

### Testing area

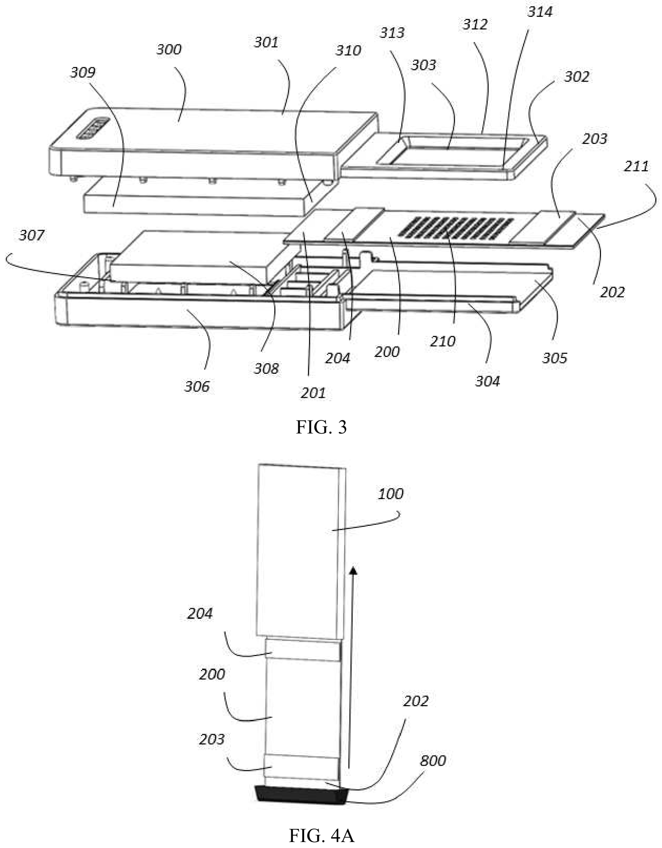

In some embodiments, the testing area is arranged on the porous membrane and used to test or assay the analyte in the liquid sample; test substances are treated on the testing area, can directly or indirectly bind to or react with the analyte in the liquid sample for testing the presence or absence or quantity of the analytes in the liquid sample. In some embodiments, one or more than two testing units are arranged on the testing area, and each testing unit can correspondingly test one analyte in the liquid sample. If a plurality of testing units are provided, a plurality of different analytes in a same liquid sample can be tested. In some embodiments, one first receptor can be immobilized onto each testing unit, and each receptor directly or indirectly binds to one analyte in the liquid sample. In some embodiments, the first receptor is an antibody or antibody fragment; alternatively, the first receptor is an antigen or an antigen fragment. These first receptors can specifically bind to the analyte in the liquid sample. If it is desired that the plurality of analytes in the liquid sample are once tested, these different first receptors are arranged on the testing area in an array, or a plurality of testing units are arranged on the testing area in an array (as shown in FIG. 4, FIG. 5A, and FIG. 5B). Such an array is that the testing units are arranged on the testing area in rows and columns; each row or column has a plurality of testing units, for example, 2-20 testing units. If there are 10-12 rows of testing units and each row has 2-20 testing units, there are 20-240 testing units in total and there are spaces between these testing units; each testing unit can be used to test one analyte in the liquid sample, and 20-240 analytes in the liquid sample can be once tested, thereby realizing a high-throughput test, as shown in FIG. 5A and FIG. 5B. These first receptors are immobilized onto the testing units. For example, one first receptor is immobilized onto each testing unit; if there are 100 testing units, 100 different first receptors are immobilized thereon; when the liquid sample flows from the upstream 5023 of the array to the downstream 2051 thereof, all these testing units contact with the liquid sample to generate reaction or binding to form different complexes. For example, if the liquid sample includes a target analyte, these first receptors capture the analyte, thus forming complexes. These complexes cannot flow with the liquid. For example, in FIG. 5A and FIG. 5B, nine rows of testing units are distributed in FIG. 5A, the most downstream row of testing units is used as a test structure control array, the leftmost column 2057 is also used as a testing result control array unit, and a testing unit array 2054 is located in the middle. If the liquid sample includes the test analyte during the test, the testing unit in the test array has test signals; as shown in FIG. 5B, the testing units 2060, 2059, 2058 on the testing area show color, indicating that the testing results are positive; the arrays 2056, 2057 in the testing result control area show positive results, indicating that the testing results of the testing units are effective. In a manufacturing process, the first receptor is dripped onto the testing area on the porous membrane with a microneedle; the first receptor is allowed to be immobilized onto the porous membrane and dried; when multiple items need to be tested, they can be simultaneously delivered to the porous membrane through a plurality of microneedles to form a testing area arranged in an array.

The testing unit is used to test the analyte in the liquid sample based on the principle of immune binding or the non-immune principle, for example, the principle of nucleic acid binding. For example, a sequence complementary to a target nucleic acid can be immobilized onto the testing unit; after the target nucleic acid is amplified, the target nucleic acid can bind to the immobilized complementary nucleic acid, and a labeled probe is added and binds to the target nucleic acid, such that nucleic acids of different targets are tested. In some embodiments, the nucleic acid principle is used to test, including but not limited to, a DNA mixture with a same sequence or complementary sequence amplified by PCR for determining DNA, and a DNA mixture with a same sequence or a complementary sequence amplified by reverse PCR for determining RNA. Further, the above-mentioned immune reaction chip element can be applied to nucleic acid hybridization experiments, and the intermediate complexes formed are porous membrane - DNA (immobilized on the membrane) - DNA (target nucleic acid) - DNA (probe) - FITC complex, DNA-DNA-Biotin complex, DNA-DNA-Bition-streptavidin-enzyme complex, and DNA-DNA-FITC-Anti-FITC antibody-enzyme.

Usually, the above application is easily realized based on the immune binding principle. Common methods include one-step sandwich, two-step sandwich, indirect method and competition method. In some embodiments, an immune method is generally that the first receptor is immobilized onto the testing unit, and can specifically bind to the analyte or the complex; and of course, the first receptor can also specifically bind to the analyte, such that the first receptor captures the complex or the analyte. Then, the analyte is allowed to specifically bind to the second receptor with the label, and the second receptor can also specifically bind to the analyte, such that the second receptor with the label is captured, and the presence or absence or quantity of the analytes can be obtained by reading the quantity or presence or absence of the labels. In some embodiments, the complex may be some intermediates after reaction, such as protein-protein complexes, protein-protein-labeled protein complexes and protein-labeled protein complexes. The protein here also means a polypeptide fragment. The labeled protein can be an enzyme or a luminescent protein. In some embodiments, according to the presence or absence of the labeled protein in the complex, when the labeled protein is absent in the formed immune complex, all liquids or liquid reagents contact with the liquid inlet area of each testing unit in the testing area, flow through the testing area unidirectionally through micropores on the porous membrane, flow out from the liquid discharge area, and are absorbed and stored by the liquid absorption material. When the complex with the labeled protein is formed, the reaction liquid is directly added to the testing area on the porous membrane, and the added reaction liquid slowly flows through the liquid discharge area and then is absorbed and stored by the liquid absorption material. Thus, the reaction liquid includes a reagent that reacts with the label, so as to generate a substance indicating the presence or absence or quantity of the analyte after the reaction; the substance can be a color substance visible to the naked eye, a non-color substance read by a machine, a luminous substance such as fluorescence, or other substances visible or invisible to the naked eye.

In some embodiments, the first receptor and the second receptor may be an antibody or antibody fragment. The first receptor is immobilized onto the testing area; if there are a plurality of different first receptors, they are immobilized in an entire array. The second receptor also includes the label, and the label here is any substance that can directly or indirectly indicate the presence or absence or quantity of the analyte, such as fluorescence, colored particles and colored dyes. Alternatively, the label reacts with the reaction liquid to generate some reaction products, which can indicate the quantity or presence or absence of the analyte; and these reaction products may be colored substance, luminescent substances or any other substances. In some embodiments, the label is an enzyme, there is a colored substance in the reaction liquid that reacts with the enzyme, and the colored substance is deposited on the testing area or the testing unit, thus indicating the presence or absence or quantity of the analyte. If the reaction product glows, the quantity of the analyte can be judged according to the intensity of luminescence.

In some embodiments, when a plurality of testing units 2060, 2059, 2058 are arranged on the testing area, each of the testing units can be used to specifically test one analyte in the liquid sample, but is often not enough for all tests. A reference testing unit can be arranged on the testing area and designed for one receptor other than a plurality of analytes; if the plurality of testing units can test the analyte or some testing units can test the analyte, the reference testing unit can still show positive results, and all testing units show negative results; if the reference testing unit shows positive results, this means the presence of the analytes, and these analytes cannot be tested by other multiple testing units. For example, when anti-allergic antibodies are tested, although there are many anti-allergic proteins or antibodies, sometimes testing units set all show negative results and the reference testing unit shows positive results. This at least indicates that anti-allergic antibodies are present in the liquid sample, but are not specifically tested in specific testing units. Of course, the reference testing unit 2017 can also be used to test a total quantity of same-type analytes. For example, when the anti-allergic antibodies are tested, the plurality of testing units correspond to specific anti-allergic antibodies, and the reference testing unit is used to test a total quantity of anti-allergic antibodies. This is also feasible.

### First receptor and second receptor

In some embodiments, one or more first receptors are immobilized onto the testing area, and can directly or indirectly bind to the analyte in the liquid sample. The first receptors here may be antibodies, antibody fragments, antigens or antigen fragments, nucleic acids or nucleic acid fragments, or polypeptide fragments. The second receptor can also specifically bind to the analyte directly or indirectly. The second receptor here can also be antibodies, antibody fragments, antigens or antigen fragments, nucleic acids or nucleic acid fragments, or polypeptide fragments. In some embodiments, the second receptor includes the label, and the label can be directly or indirectly connected or coupled with the second receptor. For example, when the antibody (the analyte) in the liquid sample is tested, the first receptor may be the antigen, and the second receptor may be the antibody of the antigen in the liquid sample. In some embodiments, the first receptor may be the first antibody of the antigen in the liquid sample, and the second receptor may be the second antibody of the antigen in the liquid sample. When the analyte in the test sample is the antigen, the first receptor can be the first antibody of the antigen, and the second receptor can be the second antibody of the antigen. These second antibodies can be connected with the label, and the label can be used as a signal substance to show the presence or absence or quantity of the analytes in the liquid sample.

### Antibody

The antibody used in the present invention can be any antibody capable to specifically bind to the analyte in the liquid sample. The antibody that can be used as a binding agent can be any antibody known to those skilled in the art. The "antibody" can be an immunoglobulin molecule and an antigen binding part of the immunoglobulin molecule, that is, a molecule including an antigen-binding site that specifically binds to an analyte, an analyte analogue or a ligand ("immune reaction"). The term "antibody" used herein also includes derivatives of antibodies in which the binding capacity is maintained, and any protein including a binding domain that is homologous or largely homologous to the binding domain of immunoglobulin. These proteins may be derived from natural substances, or may be partially or completely synthesized. An antibody may be monoclonal or polyclonal. An antibody may be a member of any immunoglobulin, including any human-derived immunoglobulin: IgG, IgM, IgA, IgD, IgG and IgE. An "antibody fragment" is a derivative of an antibody or a part of an antibody that is less than its full length. The antibody fragment can retain at least one significant site of the binding capacity of the full-length antibody. Examples of the antibody fragment include Fab, Fab', F(ab')₂, scFv, Fv, dsFv dimer, and Fd fragments, but are not limited to the above. The antibody fragment can be generated in any way. For example, the antibody fragment can be generated by enzymatic hydrolysis or chemical cleavage of a complete antibody, or by recombination from genes for which partial antibody sequences are encoded. In other words, the antibody fragment can be partially or completely reconstituted. The antibody fragment can be any single-chain antibody fragment. In other words, the antibody fragment can include multiple peptide chains that are linked to each other, for example, by disulfide bonds. The antibody fragment can also be any of multimolecular complexes. A functional antibody fragment usually includes at least about 50 amino acids, while more antibody fragments usually include at least about 200 amino acids. Single-chain Fvs (scFvs) is a recombinant antibody fragment, which consists of only variable light chain (V_{L}) and variable heavy chain (V_{H}) covalently bound to each other in a polypeptide chain. One of V_{L} and V_{H} has an amine terminal region. The length and composition of polypeptide chain are variable, and its length can cause two variable domains to be bridged and has no serious influence on the arrangement of atoms. Generally, polypeptide chain is mainly composed of glycine and serine residues, and some glutamic acid and lysine residues are scattered to increase their solubility. "Dimer" refers to the dimer of single-chain Fvs. The monomers of dimers usually include shorter peptide chains than those of most single-chain Fvs, and they show a tendency to form the dimers.

"Fv" fragment consists of one V_{H} domain and one V_{L} domain which are non-covalently linked to each other. The term "dsFv" used here refers to Fv including an intermolecular disulfide bond of a stable V_{H}-V_{L} pair. "F(ab')" fragment is an antibody fragment, and substantially the same as the fragment obtained by digesting immunoglobulin (usually IgG) with pepsin at pH of 4.0-4.5. The fragment can also be recombined and synthesized. "Fab'" fragment is an antibody fragment, and substantially the same as the fragment obtained by reducing disulfide bonds connected with two heavy chains on the F(ab') fragment. The F(ab') fragment can also be recombined and synthesized. "Fab" fragment is an antibody fragment that is substantially the same as the fragment obtained by digesting immunoglobulin (usually IgG) with papain. The Fab fragment can also be recombined and synthesized. The heavy chain fragment on the Fab fragment is the Fd fragment.

The antibody here can also be any kind of antibodies in the analyte, and these antibodies can also be used as the analyte.

### Nucleic acids

In some embodiments, the device of the present invention can be used to test the quantity, presence or absence of nucleic acids in the liquid sample in a high-throughput way. Generally, the first receptors include objective nucleic acids with substantially complementary sequences ; the sequences are immobilized onto the testing area, but the objective nucleic acids or the target nucleic acids (which can also be called analytes) exist in the liquid sample; alternatively, the amplified products of the objective nucleic acids or the target nucleic acids in the liquid sample cause the complementary sequences to bind to the objective nucleic acids or the amplified nucleic acids form a conjugate; then, a probe is allowed to bind to the objective nucleic acids or the target nucleic acids and can include the label, for example, fluorescent labels; the quantity of the objective nucleic acids can be identified through the quantity of the fluorescent labels on the probe. Amplification here may be any form of amplification, for example, PCR amplification, isothermal amplification, RPA technology or RAA technology; primers and necessary reagents to complete amplification are needed for nucleic acid amplification. All these methods are conventional technologies.

The term "substantially complementary", when used to define an amino acid sequence or a nucleic acid sequence, means that a specific target sequence such as an oligonucleotide sequence is substantially complementary to all or a part of selected sequences, and therefore will specifically bind to a part of mRNA to encode the selected sequences. Therefore, generally, the sequences will be highly complementary to the "target" sequence of mRNA, and the complementary part of the sequences will have no more than about 1, about 2, about 3, about 4, about 5, about 6, about 7, about 8, about 9, or about 10 base mismatches. In many cases, it may be desired that the sequences are exactly matched, that is, the sequences are completely complementary to those specifically binding to the oligonucleotide, so zero mismatch exists in a complementary segment. Therefore, generally, highly complementary sequences will quite specifically bind to the target sequence region of mRNA, thus effectively reducing and/or even inhibiting translation of the target sequence of mRNA into a polypeptide product.

A substantially complementary nucleic acid sequence will be more than about 80% complementary (or "% exact match") to a corresponding nucleic acid target sequence that specifically binds to the nucleic acids, and more preferably, will be more than about 85% complementary to a corresponding target sequence that specifically binds to the nucleic acids. In some aspects, as mentioned above, it will be desirable to have even more substantially complementary nucleic acid sequences in practices of the present invention; in such cases, the nucleic acid sequences will be more than about 90% complementary to the corresponding target sequences that specifically bind to the nucleic acids, and in some embodiments, the nucleic acid sequences can be more than about 95% complementary to the corresponding target sequences that specifically bind to the nucleic acids, and the nucleic acid sequences can be even up to and including about 96%, about 97%, about 98%, about 99% and even about 100% exactly complementary to all or a part of the target sequences that specifically bind to the designed nucleic acids.

The percentage of similarity or complementarity of any disclosed nucleic acid sequence can be determined, for example, by comparing sequence information using GAP computer program version 6.0 available from the University of Wisconsin Genetics Computer Group (UWGCG). Comparison methods of Needleman-Wunsch algorithm (1970) are adopted in the GAP program. In short, the GAP program defines similarity as the number of similar comparison symbols (namely, nucleotides or amino acids) divided by the total number of symbols in the shorter sequence of two sequences. The preferred default parameters of the GAP program include: (1) a unary comparison matrix of nucleotides (including a value of 1 indicating identity and a value of 0 indicating non-identity), and a weighted comparison matrix of Gribskov and Burgess (1986); (2) a penalty score for each gap being 3.0 and an extra penalty score for each symbol in each gap being 0.10; and (3) no penalty score for an end gap.

Naturally, the present invention further includes nucleic acid segments that are complementary, essentially complementary and/or substantially complementary to at least one or more specific nucleotide sequences specifically set forth herein. A "complementary" nucleic acid sequence is a nucleic acid sequence that can be subjected to base pairing according to standard Watson-Crick complementary rules. As used herein, the term "complementary sequence" means a substantially complementary nucleic acid sequence, and can be evaluated through comparison of same nucleotides described above or defined as being hybridized with one or more specific nucleic acid segments disclosed herein under more stringent conditions, such as those just described above.

As used herein, the term "virtually excluding" or "substantially excluding" in relation to the amount of components preferably mean that the composition includes a compound of less than about 10% by weight, preferably less than about 5% by weight, and more preferably less than about 1% by weight. In preferred embodiments, these terms mean less than about 0.5% by weight, less than about 0.1% by weight or less than about 0.01% by weight.

The probes and primers used in the present invention may have any suitable length. The numerical values are assigned to the sequence, for example, 1 denotes a first residue and 2 denotes a second residue, so an algorithm can be proposed to limit all probes or primers included in a given sequence: n to n+y, where n is an integer from 1 to the last number of the sequence, y is the length of the probe or primer minus 1, and n+y does not exceed the last number of the sequence. Therefore, for a probe or primer with 25 base pairs (namely, a "25-mer"), sets of probes or primers correspond to bases 1 to 25, bases 2 to 26, bases 3 to 27, bases 4 to 28, and the like over the entire length of the sequence. Similarly, for a probe or primer of 35 base pairs (namely, a "35-mer"), an exemplary primer or probe sequence includes, but is not limited to, a sequence corresponding to bases 1 to 35, bases 2 to 36, bases 3 to 37, bases 4 to 38, and the like over the entire length of the sequence. Similarly, for a 40-mer, such probes or primers can correspond to nucleotide sequences from a first base pair to a 40th base pair, from a second base pair to a 41st base pair, from a third base pair to a 42nd base pair, and the like; for a 50-mer, such probes or primers can correspond to nucleotide sequences from a first base pair to a 50th base pair, from a second base pair to a 51st base pair, from a third base pair to a 52nd base pair, from a fourth base pair to a 53rd base pair, and the like.

The term "substantially corresponding to", "substantially homologous" or "substantially identical" as used herein indicates the characteristics of a nucleic acid sequence or an amino acid sequence, where the nucleic acid sequence or the amino acid sequence selected has at least about 70% or about 75% sequence identity when compared with the reference nucleic acid sequence or the reference amino acid sequence selected. More generally, the sequence and the reference sequence selected will have sequence identity of at least about 76%, 77%, 78%, 79%, 80%, 81%, 82%, 83%, 84% or even 85%, and more preferably at least about 86%, 87%, 88%, 89%, 90%, 91%, 92%, 93%, 94% or 95%. Still more preferably, highly homologous sequences often share more than at least about 96%, 97%, 98% or 99% sequence identity between the selected sequence and the reference sequence with which it is compared.

### Array

In some embodiments, a plurality of testing units or a plurality of different first receptors are arranged on a testing area in an array; these receptors can specifically bind to complexes or analytes; and the analytes can specifically bind to a second receptor. In some embodiments, the second receptor can include a label. In some embodiments, a plurality of testing units or a plurality of first receptors are arranged on the testing area in rows. For example, the testing area is arranged in an area of a porous membrane, and the testing units in the testing area are arranged in an array. The "array" here means that a plurality of testing units are arranged in a square matrix, and such arrangement includes a single row, a single column, a plurality of rows or a plurality of columns. Alternatively, staggered arrangement is used for rows and columns. In some embodiments, there is a specified distance between the testing units; alternatively, there is a specified distance between the testing units and their surrounding testing units; alternatively, there is a specified distance or interval between the testing units. In general, the array can be any shape of square, rectangle, triangle, trapezoid, rhombus, and the like. Of course, the array can also include one testing unit; a single testing unit is only used to test one analyte in the liquid sample; and the array also belongs to a special form of an entire column. Therefore, the testing units are arranged in the testing area in an array and can detect one or more analytes in the liquid sample, for example, 2-200, 2-100, 2-50 and 2-40 analytes. These analytes can be tested simultaneously with a same sample.

In some embodiments, the testing units or the first receptors on the testing area are arranged in an array, such that the liquid can flow through each testing unit when flowing over the testing area, and the first receptors can be allowed to directly contact with the liquid sample. Then, if the analyte is present, the analyte can be allowed to directly or indirectly bind to the first receptors. Thus, the testing area is located in a liquid flowing direction.

The first receptors can be arranged on the testing area in an array through the following way: once drop a plurality of different first receptors onto the testing area with the microneedle; immobilize the first receptors onto the testing area; and then dry the first receptors immobilized. Of course, the solution of the first receptor can include any other components that help to immobilize the first receptor onto the testing area without any substantial movement. A plurality of microneedle arrays can be adopted; each microneedle is connected with a solution of a receptor; droplets are generated from the microneedle outlets and applied onto the porous membrane, such that a plurality of testing units can be once arranged or manufactured on the porous membrane.

### Liquid or solution

The liquid here is the collective term of the liquid needed to complete the test of the analyte. The liquid here has many different forms or functions; these forms or functions are required to complete the test, but are not necessarily required; they are used to obtain better test results, and belong to a preferred embodiment.

In some embodiments, the liquid of the present invention includes a liquid sample. A solid sample being dissolved into a solution is also an embodiment of a specific liquid sample according to the present invention. For example, the solid sample needs to be dissolved by a liquid solvent and diluted to become a liquid sample; generally, an aqueous liquid solvent is used for dissolution or dilution. Of course, with the sample itself being liquid, one end of the porous membrane directly contacting with the liquid sample is also a specific embodiment of the present invention. In some embodiments, even if the sample itself is the liquid sample, it is always desired that the liquid sample is treated, for example, a dilute solution is used to treat the liquid sample, or the liquid sample is diluted with the dilute solution after subjected to pretreatment such as filtration, purification and centrifugation; then, the treated liquid sample is allowed to contact with one end of the porous membrane, flow on the porous membrane, flow through the testing area and the testing result control area and flow into the water absorption area. Alternatively, when a very small amount of the liquid sample is required or the liquid sample may include a large amount of the analyte, in order to moisten the testing area on the porous membrane, the very small amount of the liquid sample can be diluted by the solution to increase the volume of the liquid sample, and the testing area can be moistened by the liquid.

Generally, during the first test, the sample solution or a solution including a sample is allowed to contact with one end of the porous membrane, and the sample solution is allowed to flow through the testing area, such that a first receptor or a plurality of different first receptors are allowed to contact with the liquid sample, for example, a first antibody contacts with the liquid sample; if an analyte exists in the liquid sample, the plurality of first receptors bind to a plurality of analytes in the liquid sample to form a binding complex immobilized onto a plurality of testing units. In this case, the dry porous membrane is moistened. If excess liquid flows through the testing area, it flows into the water absorption area and is absorbed by the absorbent material of the water absorption area after moistening the entire membrane.

In order to improve the test accuracy, after the porous membrane absorbs the liquid sample, it is desired that the moistened membrane is washed with washing liquids to wash off some other substances not bound by the first receptors, such as non-target substances; with the analyte being the antibody, some impurities can be washed off, for example, non-antibodies, as well as erythrocytes and other impurities in the blood sample. The components of the washing liquid can be some buffer reagents, and these washing liquids here are mainly to wash away impurities or interfering substances on the porous membrane, in order to strengthen the effect of next reaction. For example, after one end of the porous membrane receives the liquid sample, the washing liquids are used to wash the testing area to wash off impurities or interfering substances therein. After the testing area is washed, one end of the porous membrane contacting with the liquid is allowed to contact with a liquid including a label; the second receptor includes the label and can be captured by the complex when flowing to the testing area, and the label is immobilized onto the testing unit. In this case, the washing liquids can be used to wash off the second receptors not captured by the complex, thereby avoiding subsequent generation of false positive. The label needs to be washed if having a color signal. If it is desired to focus on testing 10 or dozens of analytes in a small testing area, there is a relatively close distance between the testing units; after the label with colored particle signals is immobilized by the testing unit, redundant labels not immobilized by the testing unit are washed away by the washing liquids, which results in more accurate test for the testing unit. In the prior art, especially when the concentration of the liquid sample is very low, or approximate to a test threshold (for example, less than the threshold, or slightly greater than the threshold), it is more important to obtain an accurate test. If the colored particles not immobilized are not washed away, false positive or false negative results are likely to occur. However, redundant free labels not immobilized by the testing unit can be washed away by the washing liquids, which can make the testing results relatively accurate. In addition, this can also make a boundary between the testing unit clear, and reading by naked eyes or machines is relatively clear and accurate. It can be understood that the types of washing reagents here can be freely selected according to specific products, and the timing or conditions in which one end of the porous membrane contacts with the washing liquid can also be freely selected according to the specific products.

In some embodiments, the moistened porous membrane can be allowed to contact with a liquid including a blocking reagent; the liquid including a blocking reagent is allowed to flow through the testing area; and the blocking reagent is used to block micropores in other areas of the porous membrane without including the first receptor. The other areas used here can be called blank areas that are pores between the testing units or between the testing result control areas or the reference testing units, which avoids background interference caused by a fact that subsequently added reagents are adsorbed or immobilized by the micropores of the porous membranes of the blank areas; alternatively, the blocking reagent is used to block the non-specific site on the testing unit, and then increase the binding specificity when the second receptor flows through the testing area. Therefore, the blocking reagent can be reagents of any composition, including reagents capable to block, occupy and fill the micropores in other blank areas on the porous membrane where the first receptors are not immobilized, or reagents to block the non-specific sites on the testing units; and these reagents are used to purify the test background and reduce the influence of the background on the testing results. However, if the testing result control area or the testing result reference area is further provided on the testing area, the liquid including a blocking reagent generally flows from one end of the porous membrane to the other end thereof, substantially covering one or more of the testing area, the testing result control area and the testing result reference area. In some embodiments, the blocking reagent includes protein, polypeptide or other substances similar to protein or polypeptide; and the blocking reagents act as their blocking function depending on the sizes of the micropores on the porous membrane. As long as the sizes of the micropores on the porous membrane are determined, protein with an appropriate size can be selected to block the micropores. It can be understood that when the first receptors need to be pretreated and immobilized on the porous membrane, these receptors have occupied one site or a plurality of micropores of the porous membrane; for example, the plurality of micropores are occupied or filled by the first receptors, the blocking reagent is mainly used to block the micropores not occupied by the first receptors, and these micropores are not selective for adsorption of substances. If many irrelevant substances are not selectively adsorbed, a later background may be deepened, and signals on the testing unit cannot be well distinguished. Of course, the blocking reagent is used to block some non-specific sites on the testing unit, such that substances subsequently bound to the testing unit are more specific.

In some embodiments, the blocking reagent is mainly casein. It can be understood that these blocking reagents are not proteins or nucleic acids or antibodies that have the same properties as the first receptors. In some embodiments, the blocking liquid can be mixed with the washing liquid to form a mixture. Of course, the washing liquid can also exist independent of the blocking liquid.

The blocking liquid, the washing liquid and the liquid sample may contact with the porous membrane in arbitrary sequence; for example, the blocking liquid is allowed to contact with the porous membrane, the sample solution is allowed to contact with the porous membrane, and then the washing liquid is allowed to contact with the porous membrane. Alternatively, the porous membrane without contacting with other solutions is washed by the washing liquid. In some embodiments, the porous membrane can be allowed to contact with the sample solution and then the blocking liquid or the mixture of the blocking liquid and the washing liquid. The embodiment is one of preferred embodiments. In some embodiments, a liquid for diluting or dissolving a liquid sample does not include the protein or the blocking reagent.

In some embodiments, after the blocking reagent or the blocking liquid contacts with the porous membrane, the blocking liquid is allowed to flow on the porous membrane under the capillary action of the water absorption area, to block the micropores or non-specific sites on the porous membrane. After the micropores or non-specific sites on the porous membrane are blocked, the solution including second receptor with a label is allowed to contact with the porous membrane, the solution including second receptor is allowed to flow through the testing area from one end of the porous membrane contacting with the liquid through the capillary force of the water absorption area, to specifically bind to the complex formed by the analyte and the first receptor. The second receptor includes labels that can generate signals, for example, optical signals, or the labels have color signals or react with the reaction liquid to generate signals. Therefore, in some embodiments, before the solutions including second receptor contacts with the porous membrane, the blocking liquid is allowed to contact with the porous membrane and flow through the testing area. Thus, the second receptor only binds to the analyte bound by the first receptor to the greatest extent, and is absorbed by the pores in other blank areas to the least extent. Of course, in some cases, neither the blocking liquid nor washing after sample contact are needed. With the label being colored particles or water-soluble pigments, the solution including second receptor contacts with the porous membrane and flows through the testing area, and the analyte captured by the first receptor on the testing area is used to capture the second receptor with the label, such that the second receptor can precipitate or be captured and immobilized by the complex. Indirectly, the colored particles or dyes also precipitate, indicating the presence or absence or quantity of analytes.

When the label included in the second receptor needs to react chemically with the reagent in the reaction liquid to generate signals, it is best to block the porous membrane in advance with the blocking reagent, such that the second receptor is allowed to bind to a complex formed by the first receptor-analyte to the greatest extent and is not absorbed by the micropores in other blank areas as much as possible. Therefore, there are a few second receptors including a label in the blank areas. However, most of the second receptors including a label are bound by the complex on the testing unit. When the reaction reagent in the reaction liquid reacts with the label, the signals are mainly generated in the area of the complex, but rarely generated in other blank areas, which reduces the interference of background signals and makes the testing results more accurate. The blank areas mentioned here refer to areas where no substance is treated, including the area between the testing units, the area between the testing result control units in the testing result control area, and the area between the testing result reference area and the testing unit; generally, the blank areas include a plurality of micropores, without the receptors.

In order to realize the test, an enzyme labeling way is adopted, and the enzyme is labeled on the second receptor. When the solution including a second enzyme-labeled receptor flows through the testing area, the testing result reference area or the testing result control area, the second receptor binds to the complex and is immobilized onto the testing unit, the testing result control unit or the testing result reference unit. The second receptor here can be an antibody, an antigen, a nucleic acid probe, or the like as defined above. When the solution including a second enzyme-labeled receptor flows through the above areas, excess liquid enables the enzyme-labeled solution to flow through the porous membrane under the capillary force of the water absorption area. This operation way is that one end of the porous membrane contacts with the liquid, allowing the liquid to flow through the above areas and then flow into the water absorption area.

In some embodiments, the second enzyme-labeled receptor is immobilized into the corresponding area; the washing liquid can be used to wash the second enzyme-labeled receptor reagent left in the reaction areas (which may be one or more of the testing area, the testing result control area, the testing result reference area and the like). These washing liquids are of a preferred scheme but not of a necessary scheme.

In some embodiments, after the second enzyme-labeled receptor is bound or captured by the reagent in the reaction area, an enzyme-labeled substrate solution or a reaction liquid is added, the reaction liquid includes a reagent reacting with the enzyme, and the reaction reagent reacts with the enzyme to generate signals; for example, the reagent in the substrate solution or the reaction liquid can be catalyzed by the enzyme to generate the signals if contacting with the enzyme-labeled immune complex, for example, visible substances, fluorescent substances or shimmering substances in visible light; these substances can be observed by naked eyes or by measuring fluorescence and shimmering signals with sensors.

### Flowing of solution or liquid and porous membrane

In some embodiments, the above liquids, such as liquid samples, washing liquids, solutions including second receptor with a label, and other washing liquids, can repeatedly contact with one end of the membrane and flow through the area on the membrane through the capillary force of the water absorption area at the other end of the membrane, thereby conducting multiple different reactions and implementing the test of analytes. For example, the first receptor immobilized binds to the analyte form a complex, and then the second receptor with the label is captured by the complex and immobilized onto the membrane. When the label needs to contact with the reaction liquid to react, the reaction liquid needs to flow through the membrane such that the reaction liquid reacts with the label to generate signals, for example, signals visible to the naked eye (such as color signals), or signals read by equipment (such as fluorescence signals). The color signals here can be color precipitates, precipitates of colored substances or any other forms of visible signals. The reaction liquid can also be sucked onto the porous membrane from one end of the porous membrane contacting with the liquid sample, flows through the testing area to implement contact reaction with the label, and finally flows through the water absorption area.

The above liquids flow from upstream to downstream, for example, from one end of the porous membrane contacting with the liquid (upstream, namely, the liquid sample contact area 202) to the testing area 2001, and then to the testing result control area or the testing result reference area; the testing units of the testing areas are arranged on the porous membrane in an array; generally, the array close to the upstream earlier contacts with the liquid than the array far away from the upstream; in other words, the area close to the water absorption area later contacts with the liquid than the array close to the upstream.

Specific micropores and thicknesses of porous membranes need to be used for different labels, analytes, and first and second receptors, thereby ensuring that different kinds of liquids can flow through the membranes many times and implementing necessary reactions in a flowing process. It can be understood that some reactions require a long time, and others require a short time, which can be effectively selected through specific experiments based on the thicknesses of the porous membranes or/and the sizes of the micropores and can be freely realized by persons skilled in the art by reading the description of the present invention.

In some embodiments, the appropriate porous membrane is selected according to the size of the component of the analyte. For example, if there are a plurality of first receptors, the first receptors are immobilized onto the testing area in an array. If many kinds of analytes exist in the liquid sample and the relatively large molecular weight, it takes a long time to implement contact reaction between the analytes and the first receptors and the membrane with the small pore size can be selected, such that the liquid sample can flow slowly on the membrane, and the first receptors can be allowed to have sufficient time to contact with the liquid sample and can fully capture the analytes in the liquid sample.

In addition, in other some embodiments, if there are many analytes to be tested once, such as 20-50 analytes to be tested once, it is also desired that the flow rate of the liquid slows down, and more than 20-50 testing units are allowed to contact with the liquid sample for a long time, such that each testing unit can contact with the liquid sample as much as possible to fully capture the analytes. In this case, the molecular weight or spatial structure of the analyte may be very small, such as small chemical molecules; the porous membrane with the small pore size is selected, such that the flow rate of the liquid slows down to implement liquid reaction during flowing.

In other embodiments, when the second receptor with a label contacts with the porous membrane in a liquid form, the volume or molecular weight of the label is also related to the pore size of the porous membrane; when the label is in a form of particles, the sizes of common particles may be smaller than the sizes of the micropores or equivalent to the sizes of the micropores on the membrane, such as nano-scale particles, gold particles and latex particles. The second receptor with a label can flow through the testing area in case of few analytes. If a plurality of testing units (for example, 10 or more than 20 testing units) exist, the nano-scale particles may block the micropores of the porous membrane, thus causing the liquid not to effectively flow through the testing units and affecting the test accuracy. In this case, it is necessary to select the porous membrane with the size much larger than the size of the label. Thus, the second receptor including the particles can be effectively captured by the complex (the first receptor-analyte) and precipitate when the liquid flows through the porous membrane.

Generally, the enzyme as the label is in a form of protein and has the relatively large molecular weight and particle size, but is far smaller than the size of the porous membrane, without blocking the micropores of the porous membrane. If the enzyme is regarded as a sphere, the size of the labeled enzyme is related to the molecular weight of the enzyme itself. For example, horseradish peroxidase (HRP) has a molecular weight of approximately 40kD and a molecular diameter of approximately 4nm; and recombinant alkaline phosphatase (AP) has a molecular weight of approximately 80kD and a molecular diameter of approximately 5nm. If the immune complex exists in a form of membrane-first receptor-analyte-second receptor-enzyme, the thickness of the analyte-second receptor-enzyme relative to the membrane-first receptor in the immune complex is only increased by approximately 16nm and much smaller 8µm (8,000nm) than the pore size of the porous channel on the membrane, and the enzyme-labeled immune complex generated will not block the internal channel of the membrane or prevent the solution including a second enzyme-labeled receptor from continuing to diffuse backwards and downstream to make immune reaction with binding sites of other downstream or subsequent testing units; therefore, the solution including a second enzyme-labeled receptor is captured and immobilized by other downstream testing units.

For the membrane with the pore size of 8µm as mentioned above, the pore size is measured by an electron microscope. In practical use, the micropore size of the membrane can be judged according to the lateral flow rate of the liquid in the membrane. The pore size of the membrane is inversely proportional to the lateral diffusion time of the liquid in the membrane per unit distance. The large pore size of the porous membrane indicates the fast flow rate of the liquid, and the small pore size thereof indicates the slow flow rate of the liquid. For example, the liquid in the porous membrane with the pore size of 8µm diffuses 4cm laterally, which takes about 140 seconds. The actual diffusion time being less than 140 seconds indicates that the pore size of the porous membrane is less than 8µm and the pore size of the porous membrane is more than 8µm.

In short, the flow rate of the liquid is largely related to the property of the substance included in the liquid itself and the material of the porous membrane, especially for the size of the porous membrane. An appropriate porous membrane can be screened through limited experiments to implement an entire testing process. The entire testing process can be that the liquid sample is allowed to contact with the liquid sample contact area 202, flow through the testing area and flow into the water absorption area 100; and then the solution including second receptor with a label can be allowed to contact with the liquid sample contact area 202, flow through the testing area and flow into the water absorption area 100. Of course, in these reactions, the washing liquid can be added when washing is performed once ore more than twice; then, the reaction liquid with the label is allowed to contact with the liquid sample contact area 202, flow through the testing area and flow into the water absorption area 100. Of course, it can be understood that the reaction liquid is not necessary. For example, if the label itself is colored, the reaction liquid, such as water-soluble dyes, gold particles or latex particles, may not be needed.

### Liquid blocking element

Generally, when many kinds of liquids flow on the porous membrane, the analyte can be bound or captured, or the second receptor with a label can also be bound during the liquid flow. If the label needs to contact with the reaction liquid to complete reaction to generate detection signals, the reaction liquid can react with the label in the flowing process; the sample solution, the blocking liquid, the washing liquid and the second receptor with the label can all be received by one end of the porous membrane, and then flow through the testing area through the capillary force, to complete the assay of the analyte.

Especially, in the present invention, the porous membrane is allowed to contact with the liquid sample, the washing liquid and the solution including second receptor with a label; the liquid sample, the washing liquid and the solution including second receptor with a label can flow laterally to the water absorption area through one end of the porous membrane 200 via the capillary force to complete the whole test. In some cases, when the reaction liquid needs to react with the label, the reaction liquid can be allowed to contact with one end 200 of the porous membrane and flow laterally to the water absorption area through the capillary force, such that the reaction substance in the reaction liquid can react with the label to generate signals indicating the quantity of the analytes. However, sometimes, some reactions require long contact time, such as contact for 1-30 minutes, and the flow rate of the liquid may be relatively fast only depending on the capillary force of the water absorption area; in case of short reaction time, insufficient reaction will occur, thereby resulting in insufficient reaction results and ultimately affecting the testing results. In other words, it is impossible to obtain better test results through necessary reactions when the solution flows on the porous membrane. Once the product is assembled, although the porous membrane has been optimized and reverse sides thereof have been improved, it is still hoped that the solution will stay in the testing area more and make a full reaction.

Especially, when there are a large number of testing units in the testing area, it is not enough to complete reaction between the reaction liquid and the label to generate signals only relying on the time when the liquid flows through the testing area on the porous membrane under the capillary action, or it is not enough to consume the label immobilized by the testing units during the flow of the reaction liquid, thus ultimately affecting the test accuracy. Especially, when there are a lot of analytes to be tested, and the testing area on the porous membrane almost occupies most of the area of the whole porous membrane, it takes at most 30 seconds to 1 minute for the reaction liquid to flow from one end to the other end, and for example, at most 2 minutes when the length of the porous membrane is 2cm. In this case, it is hoped that the liquid will stay on the porous membrane for a specified time, especially on the testing area on the porous membrane, the components in the liquid are allowed to fully react with the substances on the testing unit to obtain accurate results, and the stay time is expected to be more than two minutes, for example, 2-30 minutes. For example, the substances in the reaction liquid are allowed to fully contact with the label to make a full reaction and substantially consume the label immobilized by the testing unit, such that generated signals can accurately reflect the quantity of the analytes.

Therefore, in some embodiments, the device further includes a liquid blocking element for blocking the liquid, and the liquid is allowed to stay on the porous membrane for longer or more time, especially on the testing area, such that the substances in the liquid (such as reaction liquid) are allowed to fully contact with substances on the porous membrane (label immobilized on the testing unit) and then react with such substances. The longer time here is relative and means that in absence of the liquid blocking element, the liquid normally flows through the porous membrane with a specified flow rate, and the spent time is a fixed time. However, the flow rate of the liquid slows down through the liquid blocking element, and the spent time is increased, such that there is more time for the effective components in the liquid to stay in the areas on the porous membrane, such as the testing area, the testing result reference area or the testing result control area. In particular, the reaction liquid is allowed to fully contact with the label immobilized onto the testing area for effective reaction.

In some embodiments, the liquid blocking element is a non-absorbent element and distributed around the testing area on the porous membrane. As shown in FIG. 1A - FIG. 1C, the liquid blocking element 204 is arranged on one end of the porous membrane and between the downstream of the testing area and the water absorption area and covers the porous membrane; the porous membrane is made of a flexible material; the liquid blocking element is made of a non-hygroscopic material, and has a specified weight and a specified pressure to the porous membrane, such that the thickness of the porous membrane where the liquid blocking element covers the porous membrane is slightly reduced, directly affecting the absorption capacity of the water absorption area to the liquid on the porous membrane. Normally, the flow rate of the liquid flowing through the upstream of the testing area slows down due to the weakened absorption capacity, and the flow rate of the liquid flowing through the testing area slows down, such that the liquid can be allowed to contact with the testing area for a longer time.

In some embodiments, the liquid here includes the reaction liquid, the reaction liquid is allowed to stay more on the testing area; specifically, the substances in the reaction liquid react with the label on the testing area. In some embodiments, when the reaction liquid contacts with one end of the porous membrane, the reaction liquid is allowed to flow from one end of the porous membrane to the water absorption end through the capillary force of the water absorption area at the other end and then flows through the testing area, and the liquid blocking element is arranged near the water absorption area and on the porous membrane. Thus, when the porous membrane contacts with the liquid, the liquid can be allowed to flow into the water absorption area more slowly due to arrangement of the liquid blocking element and can stay on the porous membrane for a longer time. It can be easily understood that in absence of the liquid blocking element, the water absorption area is normally connected with the porous membrane; the liquid blocking element being arranged on the porous membrane is equivalent to applying a pressure to the porous membrane, and one area of the porous membrane can be tightly pressed through the pressure to weaken and block the capillary action of the water absorption area to absorb the liquid on the porous membrane and increase the obstruction of the liquid flowing into the water absorption area, such that the flow rate of the liquid normally flowing into the water absorption area slows down and the time when the liquid stays on the porous membrane is relatively long, and the liquid can fully stay in the testing area on the porous membrane for a more sufficient reaction. It can be understood that these liquid blocking elements do not absolutely 100% restrict the flow of the liquid on the porous membrane, but slow down the flow rate of the liquid. When the porous membrane contacts with the liquid sample, the porous membrane is almost completely moistened and substantially loses the capillary force, and the water absorption property thereof is substantially absent. In this case, the subsequent liquid can continue to flow on the porous membrane mainly depending on the capillary force of the water absorption area. If the flow rate of the liquid is to slow down, the liquid blocking element is substantially arranged between the water absorption area and the porous membrane to slow down the flow rate of the subsequent liquid and prolong the time when the liquid stays on the porous membrane through any known way. The liquid blocking element is arranged in a direction where the liquid flows forward at an upper limit flow rate, and can be understood to have the function like a "dam" to block the flow of flood, such that water can gather in one place to slow down the flow rate thereof.

In some embodiments, the liquid blocking element is arranged to intersect with a liquid flowing direction, rather than parallel to the liquid flow direction. For example, as shown in FIG. 1A - FIG. 1C, the first liquid blocking element 204 is arranged near the water absorption area and on the porous membrane, and the second liquid blocking element 203 is arranged downstream of the liquid sample contact area 202. The first liquid blocking element 204 is parallel to the second liquid blocking element 203, but both the first liquid blocking element and the second liquid blocking element are perpendicular to the liquid flowing direction; in addition the testing area is located between the first liquid blocking element and the second liquid blocking element. In such embodiments, only the first liquid blocking element 204 may be included, and the second liquid blocking element may not be included. Generally, the liquid blocking element can cover the surface of the porous membrane normally, without substantially affecting the thickness of the porous membrane; a specified pressure can be applied to the liquid blocking element to reduce the flow rate of the liquid from the porous membrane to the water absorption area. The liquid blocking element may be coated on the surface of the liquid blocking element through a glue and then covers the surface of the porous membrane, and the liquid blocking element can be immobilized onto the surface of the porous membrane through the glue. Of course, the liquid blocking element can be immobilized onto the surface of the porous membrane directly through an external pressure instead of the glue. When the second liquid blocking element 203 is used, the second liquid blocking element is used to substantially separate one end of the porous membrane contacting with the liquid from the testing area, and one purpose is to hope that the liquid to be contacted substantially flows through the porous membrane, thereby preventing the liquid from flowing from the surface of the porous membrane as much as possible. Thus, the liquid receiving area is arranged upstream of the second liquid blocking element 203 and often needs to contact with many kinds of different liquids continuously, and sometimes the volumes of the liquids are relatively large. The second liquid blocking element can allow the liquid to substantially flow through the porous membrane covered by the liquid blocking element to the downstream testing area, thereby reducing the direct flow of the liquid from the porous membrane and avoiding the high flow of the liquid. In addition, as mentioned above, the liquid blocking element itself may apply pressure to the porous membrane, also allows the liquid to flow into the downstream testing area from the liquid sample contact area 202 at a slow flow rate, thus slowing down the flow rate of the liquid. With the liquid blocking element being pasted on the porous membrane, in fact, the pores of the porous membrane are also sealed by the glue, which reduces the capillary force and allows the liquid to flow onto the porous membrane. In this way, a plurality of testing units on the porous membrane can be allowed to contact with various liquids at a slow flow rate to make a full reaction.

When the reaction liquid is applied, the reaction liquid can be allowed to contact with the liquid sample contact area 202 and flow through the porous membrane and the testing area. In another embodiment, the reaction liquid 900 can also be directly dropped onto the surface of the porous membrane in a manner similar to that shown in FIG. 4B, and a layer of the reaction liquid is covered on the porous membrane. Because the liquid blocking element has a specified thickness, the reaction liquid can have a specified height on the porous membrane, and generally, the reaction substance in the reaction liquid is excessive. In this case, because the water absorption area still maintains the capillary force on the porous membrane, the liquid on the porous membrane can still be attracted or absorbed, the liquid on the porous membrane continues to be allowed to flow into the water absorption area through the capillary force, and the reaction liquid on the porous membrane reacts with the label on the testing unit, such that the reaction liquid can be allowed to stay on the porous membrane for enough time until the reaction liquid completely reacts with the label. The reaction is completed until the reaction liquid is completely absorbed by the water absorption area, that is, the reaction liquid contacts with the label to make a reaction to generate signal substances when being absorbed by the water absorption area at one end, such that the reaction liquid can be kept for a period of time. A short time here can be 30 seconds, 1 minute, 2 minutes, 3 minutes, 5 minutes, 10 minutes, 15 minutes, 20 minutes and 30 minutes. The stay time of the liquid depends on three important factors: the volume of the applied liquid, whether the liquid blocking element applies enough pressure to the porous membrane, and the water absorption capacity of the water absorption area. For example, the liquid blocking element can have a thickness, and the thickness or height thereof can be adjusted, such that the volume of the reaction liquid can be controlled. When the area defined by the porous liquid blocking element on the porous membrane is fixed, the height of the reaction liquid can be adjusted normally.

In some embodiments, the liquid blocking element is used to enclose a structure similar to the reservoir on the porous membrane; the liquid blocking element is used to enclose an area on the surface of the porous membrane; and the area includes one or all of the testing area 205, the testing result control area, and the testing result reference area therein. For example, as shown in FIG. 2A, the liquid blocking element covers the surface of the porous membrane 200 in a rectangular frame, forming a recessed area or an area 2061 similar to a liquid storage pool; the thickness of the frame is regarded as the height of water storage; the porous membrane is located at the bottom of the frame; and the other end of the frame connected with the porous membrane is the water absorption area 100 without the absorbent material therein. The reaction liquid can be directly applied in the reservoir and dripped into the area 2061, can directly diffuse in the area, and has a specified height on the porous membrane, such that the reaction liquid can fully react with the label to generate the signal substances. The signals can be read, and the term "reading" here can be implemented through direct observation by naked eyes or by any equipment. For example, reading by naked eyes is to observe the color change of the testing unit or whether color substances precipitate, and generally, electronic signals or fluorescence signals are read by the equipment.

In some embodiments, labels are enzymes, including but not limited to, HRP (horse radish peroxidase) and alkaline phosphatase; or labels are some small molecular compounds, including but not limited to, luminol, acridinium ester, fluorescein, rhodamine, and oxidant. The resulting signals can be light absorption signals of visible lights, chemiluminescent signals, and fluorescent signals. For example, reaction liquids that interact with HRP and can generate chromophore deposition signals include 3,3',5,5'-tetramethylbenzidine, 5-bromo-4-chloro-3-indolyl phosphate, tetranitroblue tetrazolium chloride, luminol, adamantane, acridinium ester, and the like; some reaction liquids may react with HRP; some reaction liquids may react with alkaline phosphatase; and some reaction liquids are alkaline solutions.

In some embodiments, the liquid blocking element is not arranged on the porous membrane separately, but depends on the window opened by the upper strip; the grid of the window covers the surface of the porous membrane, such that the liquid blocking element can also be formed separately. Of course, the grid of the window opened by the upper strip can cover the surface of the liquid blocking element forming the reservoir, which applies a specified pressure to the liquid blocking element. In addition, the grid can be combined with the liquid blocking element to form the structure of the reservoir. Therefore, the liquid blocking element is generally made of the non-absorbent material or may be made of plastic, metal, alloy, and the like. This can be used as a specific embodiment of the present invention. The element may be in a form of sheet or strip.

For example, as shown in FIG. 3, a testing area 210 is arranged on the porous membrane 200; the first liquid blocking element 203 covers one end of the porous membrane; the second liquid blocking element 204 is arranged near the water absorption area; and the testing area is located on the porous membrane between the first liquid blocking element and the second liquid blocking element and provided with a plurality of testing units 210 therein. The porous membrane covers a hard substrate 211, and then the hard substrate and the porous membrane are together placed in the slot 305 of the lower strip. Both the porous membrane and the filter paper are arranged in the chamber defined between the upper strip 300 and the lower strip 306. The chamber for accommodating the water absorption area is arranged between the upper strip 300 and the lower strip 306 and an area for accommodating the absorbent material is arranged in the chamber. In a specific embodiment as shown in FIG. 3, two water absorption elements 308, 309 are stacked together in a chamber 307 formed by the upper strip and the lower strip, where the lower water absorption element 308 is located in the chamber of the lower strip, and the upper water absorption element 309 is longer than the lower water absorption element 308; the elongated end 310 contacts with one end 201 of the porous membrane; the window 303 is opened on the upper strip and composed of rectangle-like grids 302, 313; the grids distributed in front and back cover the surfaces of the liquid blocking elements 203, 204; and the grids 312, 314 longitudinally distributed cover the porous membrane twice, such that the structure of one reservoir is formed around the testing area 210 on the porous membrane. Although the grids 312, 314 longitudinally distributed do not directly contact with the surface of the porous membrane, there is a small distance between the grids and the surface of the porous membrane, and the liquid substantially does not leak due to the surface tension of the liquid. Therefore, when directly delivered to the testing area through the window 303, the liquid directly diffuses in the testing area and forms a liquid level with a specified height, such as the reaction liquid, such that the liquid covers the testing area. In addition, because there is a large amount of the liquid, all the labels immobilized onto the testing area can react completely. During the reaction, the liquid can still penetrate through the porous membrane covered by the first liquid blocking element 204 and be absorbed by the absorbent material connected with the porous membrane. When the reaction liquid is completely absorbed, the whole reaction will be implemented, that is, the labels immobilized on the testing unit are completely reacted. In the embodiment, in fact, the grids 312, 314 of the window also function as the liquid blocking element. After the reaction, the testing results on the testing area can be read through the window 303. As described above, when the upper strip and the lower strip are assembled, the combination thereof enables the grids of the window to apply a pressure to the liquid blocking element, thereby applying a pressure to the porous membrane. In some embodiments, it is also possible that the grids 302, 313 of the window or four grids 302, 314, 312, 313 directly cover the porous membrane, instead of the liquid blocking element 203, 204 being arranged on porous membrane. However, this increases the difficulty for the processing technology of the window, and the surface that needs to contact with the membrane is very smooth whether how much pressure is applied. The embodiment is a feasible embodiment, rather than a preferred embodiment. Of course, the embodiment can also be as shown in FIG. 4A and FIG. 4B, where the whole porous membrane is exposed. In this case, the liquid blocking elements 204, 203 can directly cover the porous membrane 200, and the reaction liquid 900 can be directly dripped on the testing area, such that the reaction liquid is allowed to contact with the testing area for a long time to make a full reaction through the liquid blocking elements.

In another embodiment, the testing area is enclosed into one reservoir through the liquid blocking elements of the porous membrane; during the test, when required to contact with the testing area for a long time, the solution can be directly added into the reservoir, for example, the sample solution, the washing liquid, the blocking liquid, the solution including second receptor with a label, the reaction liquid reacting with the label or any other solutions beneficial to testing. Therefore, the liquid sample contact area 202 can be directly allowed to contact with the liquids that do not require a special reaction time, such that the liquids flow through the porous membrane covered by the liquid blocking element through the capillary force of the porous membrane or the water absorption area to implement a reaction. When such reaction requires a long time, the solution can be directly added to an area surrounded by the liquid blocking element, diffuses quickly in the area, and then moistens the whole porous membrane, such that the testing units in the testing area contact with the solution to make reaction almost at the same time. With the reaction, the capillary force in the water absorption area will continue to absorb water on the porous membrane, and the solution will be gradually absorbed, such that the solution will flow through the porous membrane similarly to make a full reaction. In some embodiments, if the label is the enzyme, the reagent solution reacting with the enzyme can be directly added to the reservoir for reaction. As shown in FIG. 6, other structures are the same as those shown in FIG. 3, but the difference is that the liquid blocking element on the porous membrane is a frame structure 20 and covers the porous membrane. The liquid blocking element is used to enclose one reservoir where the liquid can be accommodated; the porous membrane is arranged at the bottom of the reservoir; and the grid of the window of the upper strip covers the surface of the liquid blocking element 20.

### Embodiments

The following describes how the device of the present invention achieves the purpose of the present invention with reference to the specific embodiments. This is only an example to describe how the technical solution of the present invention is achieved, but does not impose any restriction on the present invention; and the specific scope is subject to the claims.

### Embodiment 1: Immune reaction chip for lateral diffusion test is used to quantitatively test visible light signals of IgM antibodies

A micro-applicator (Hangzhou Bufeng Technology Co., Ltd.) was used to drip specific antigens of CMV, HSV1, HSV2, RV and TOX (purchased from Fapon Biotech Inc.) onto the reaction area of the porous membrane of the immune reaction chip (the nitrocellulose membrane was used here, where the pore size of the membrane was 10µm, the width of the membrane was 13mm and 40mm, the thickness of the liquid blocking element was 0.8mm, and a hard substrate was arranged at the bottom of the membrane), the concentrations thereof were 0.2mg/mL and 50nL/site respectively; the distance between each testing unit or each antigen site was 3mm; and the testing unit or the antigen site was dried for 2 hours at 45°C . The membrane of the immobilized antigen and the water absorption element were together loaded into a plastic shell, such that the water absorption element was connected with the porous membrane. A solid-phase chip reaction unit including a liquid sample contact area 202, a reaction area 205 and a liquid discharge area 100 was prepared (see FIG. 3 for details).

For the convenience of expression, the liquid involved in the immune reaction was numbered by Arabic numerals:
Liquid #1: a recombinant specific anti-CAM IgM antibody, a recombinant specific anti-HSV1 IgM antibody, a recombinant specific anti-HSV2 IgM antibody, a recombinant specific anti-RV IgM antibody, and a recombinant specific anti-TOX IgM antibody (purchased from Fapon Biotech Inc.) were diluted with a pH7.4 phosphate buffer including 10mg/mL casein, and prepared into different concentrations of sample solutions at the dilution ratios of 500, 2000, 8000 and 32000.
Liquid #2: pH7.4 phosphate buffer including 10mg/mL casein.
Liquid #3: HRP (horseradish peroxidase) labeled working solution including specific anti-human IgM.
Liquid #4: pH7.4 phosphate buffer including 1mg/mL Tween 20.
Liquid #5: pH7.4 phosphate buffer including 1mg/mL Tween 20.
Liquid #6: reaction liquid (Hangzhou Bufeng Technology Co., Ltd.) including 3,3',5,5'-tetramethylbenzidine.

The liquid sample contact area 202 of the immune reaction chip element was sequentially in contact with liquid #1 (sample solution), liquid #2 (blocking liquid), liquid #3 (solution including second receptor ), liquid #4 (washing liquid), and liquid #5 (washing liquid) for 5 minutes, respectively, where the volume of each liquid was 5mL. After contacting with the liquid sample contact area 202, different liquids diffused through the testing areas of the porous membrane through microporous channels on the nitrocellulose membrane via the surface tension between the liquids and the microporous channel and the capillary driving force of the liquid absorption material in the water absorption area.

After liquid #5 ended up its contact, 200µL of the reaction liquid #6 was directly dripped onto the reaction area 205 and covered the whole reaction area. As the reaction liquid was blocked with the liquid blocking element on a complex structure membrane, the reaction liquid could slowly diffuse out of the liquid discharge area only through the microporous channels inside the membrane under the capillary action, and be retained by the liquid absorption material contacting with the reaction liquid; in this period, dot signals appeared in the reaction area until a substrate solution was completely drained after 10 minutes, and generated signals no longer changed significantly. Images were photographed by an industrial microscope.

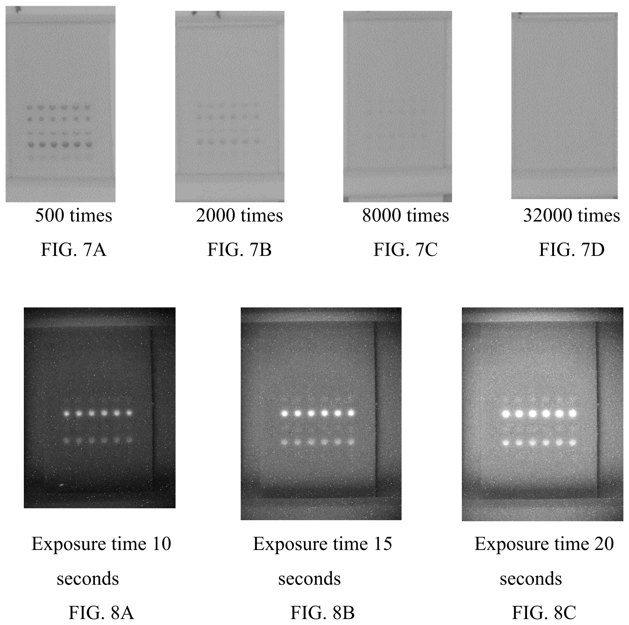

Photographed testing results are as shown in FIG. 7A- FIG. 7D. In a suitable concentration scope, specific IgM antibodies of CMV, HSV1, HSV2, RV and TOX specific antigens can be detected. Photographed testing results are as shown in FIG. 7A- FIG. 7D. The intensity of the observable signal is positively correlated with the concentration of each analyte in the liquid sample, indicating that the immune reaction chip element can also be used to quantitatively test the analyte in the liquid sample. FIG. 7A shows a sample diluted 500 times; FIG. 1B shows a sample diluted 2000 times; FIG. 7C shows a sample diluted 8000 times; and FIG. 7D shows a sample diluted 32000 times. From left to right, color on the testing unit changed from dark to light.

Because the main reaction of the present invention is achieved on the porous membrane, the continuous capillary force is applied to the porous membrane through the absorbent filter paper on the water absorption area, such that different liquids flow through the testing area of the porous membrane to complete the whole testing process. The device itself has unidirectional diffusibility and liquid storage capacity similar to those of an electronic diode, such that various mechanical modules (liquid path module, mechanical vibration and moving device, magnetic separation and cleaning module, reaction cup storage and transfer module, liquid waste disposal system, self-cleaning system, and the like) required by the current mainstream magnetic particle chemiluminescence are not needed to complete the whole immune reaction, the full-automatic determination can be simplified and the energy consumption can be minimized. An optical signal detector can be a photomultiplier tube (in this case, a direct luminescent label such as acridinium ester can also be used to further simplify testing steps), or a CCD (charge-coupled device) or CMOS (complementary metal-oxide-semiconductor transistor). In addition, as multiple substances in the chip of the present invention can be determined simultaneously, multiple items can be determined simultaneously and different items can be arbitrarily combined. This greatly improves the testing efficiency. In this case, the CCD or CMOS is usually refrigerated at ultra-low temperature. In addition to the chip structure and using method thereof disclosed by the present invention, more accurate results can be obtained using the prior art. In addition, such a testing device is also particularly suitable for home self-test, and can test multiple indicators once, and any indicator can be arbitrarily combined, such as multiple antigen indicators and multiple antibody indicators.

### Embodiment 2: Immune reaction chip for lateral diffusion test is used to test chemiluminescence signals of specific IgM antibodies of five antigens

A micro-applicator (Hangzhou Bufeng Technology Co., Ltd.) was used to drip specific antigens of CMV, HSV1, HSV2, RV and TOX (purchased from Fapon Biotech Inc.) onto the porous membrane of the immune reaction chip (the nitrocellulose membrane was used here, where the pore size of the membrane was 8µm, the width of the membrane was 13mm, and the thickness of the liquid blocking element was 0.8mm), the concentrations thereof were 0.2mg/mL, 0.2mg/mL, 0.05mg/mL, 0.2mg/mL, 0.05mg/mL and 50nL/site respectively; and such specific antigens were dried for 2 hours at 45°C. The membrane of the immobilized antigen and the water absorption element were together loaded into a plastic shell. A solid-phase chip reaction unit including a liquid sample contact area, a reaction area and a liquid discharge area was prepared (see FIG. 3 for details).

For the convenience of expression, the liquid involved in the immune reaction was numbered by Arabic numerals:
Liquid #1: a recombinant specific anti-CAM IgM antibody, a recombinant specific anti-HSV1 IgM antibody, a recombinant specific anti-HSV2 IgM antibody, a recombinant specific anti-RV IgM antibody, and a recombinant specific anti-TOX IgM antibody (purchased from Fapon Biotech Inc.) were diluted with a pH7.4 phosphate buffer including 10mg/mL OsrHSA and 10mg/mL Tween 20, and prepared into different concentrations of sample solutions at the dilution ratio of 3000.
Liquid #2: pH7.4 phosphate buffer including 10mg/mL casein.
Liquid #3: pH7.4 phosphate buffer including 10mg/mL casein.
Liquid #4: enzyme working solution including a specific anti-human IgM-HRP (horseradish peroxidase) conjugate.
Liquid #5: pH7.4 phosphate buffer including 1mg/mL Tween 20
Liquid #6: pH7.4 phosphate buffer including 1mg/mL Tween 20
Liquid #7: reaction liquid (Hangzhou Bufeng Technology Co., Ltd.) including 3,3',5,5'-tetramethylbenzidine.

The liquid sample contact area 202 of the solid-phase chip reaction chip unit was sequentially in contact with liquid #1, liquid #2, liquid #3, liquid #4, liquid #5, and liquid #6 for 5 minutes, respectively. After contacting with the liquid sample contact area, different liquids diffused through the reaction area of the solid-phase chip reaction unit through microporous channels on the nitrocellulose membrane via the surface tension between the liquids and the microporous channels and the capillary driving force of the liquid absorption element on the liquid discharge area. After liquid #6 ended up its contact, 200µL of liquid #7 was directly added to the reaction area 205, and the images at different exposure times were read by a CCD. Images read by the CCD at normal temperature and different exposure times are shown in FIG. 8.

Due to the fast and glow characteristics of enzymatic chemiluminescence, chip images photographed by the CCD at normal temperature and different exposure times show that the observable signals of analytes can be the chemiluminescence signals, which lays the foundation for the construction of a new chemiluminescence technology platform and indicates the feasibility of quantitative detection by a chemiluminescent immunoassay. FIG. 8A - FIG. 8C show the intensity of chemiluminescent signals after exposure.

### Embodiment 3: Comparative test of addition position of substrate solution or reaction liquid

### I. Addition position

The flow restriction of the liquid blocking element plays a role in controlling the constant volume of the reaction liquid in the reaction area, to ensure that all immune complexes with enzymes in the reaction area simultaneously contact with a substrate (reaction reagent) and generate observable signals. The changes in the signal intensities of the protein binding sites (testing units) where the reaction liquid contacts with the liquid sample contact area 202 and the substrate solution is directly added into the reaction area are compared to illustrate the advantage that the substrate solution is directly added into the reaction area.

A micro-applicator (Hangzhou Bufeng Technology Co., Ltd.) was used to drip specific antigens of CMV, HSV1, HSV2, RV and TOX (purchased from Fapon Biotech Inc.) onto the porous membrane of the immune reaction chip (the nitrocellulose membrane was used here, where the pore size of the membrane was 10µm, the width of the membrane was13mm, and the thickness of the liquid blocking element was 0.8mm), the concentrations thereof were 0.2mg/mL and 50nL/site respectively; and such specific antigens were dried for 2 hours at 45°C . The membrane of the immobilized antigen and the water absorption element were together loaded into a plastic shell. A solid-phase chip reaction unit including a liquid sample contact area, a reaction area and a liquid discharge area was prepared.

For the convenience of expression, the liquid involved in the immune reaction was numbered by Arabic numerals:
Liquid #1: a recombinant specific anti-CAM IgM antibody, a recombinant specific anti-HSV1 IgM antibody, a recombinant specific anti-HSV2 IgM antibody, a recombinant specific anti-RV IgM antibody, and a recombinant specific anti-TOX IgM antibody (purchased from Fapon Biotech Inc.) were diluted with a pH7.4 phosphate buffer including 10mg/mL OsrHSA and 10mg/mL Tween 20, and prepared into sample solutions at the dilution ratio of 3000.
Liquid #2: pH7.4 phosphate buffer including 10mg/mL casein.
Liquid #3: pH7.4 phosphate buffer including 10mg/mL casein.
Liquid #4: enzyme working solution including a specific anti-human IgM-HRP (horseradish peroxidase) conjugate.
Liquid #5: pH7.4 phosphate buffer including 1mg/mL Tween 20
Liquid #6: pH7.4 phosphate buffer including 1mg/mL Tween 20
Liquid #7: reaction liquid (Hangzhou Bufeng Technology Co., Ltd.) including 3,3',5,5'-tetramethylbenzidine.

The liquid sample contact area of the solid-phase chip reaction unit was sequentially in contact with liquid #1, liquid #2, liquid #3, liquid #4, liquid #5, and liquid 6# for 5 minutes, respectively. After contacting with the liquid sample contact area, different liquids diffused through the reaction area of the solid-phase chip reaction unit through microporous channels on the nitrocellulose membrane via the surface tension between the liquids and the microporous channels and the capillary driving force of the liquid absorption element on the liquid discharge area. After liquid #6 ended up its contact, the liquid sample contact area 202 contacted with 200µL of liquid #7 (Mode 1) or 200µL of the substrate solution was directly added into the reaction area (Mode 2), and then the changes in the observable signals at various sides of the reaction area under two modes were photographed by an industrial microscope at 10, 15 and 20 minutes respectively.

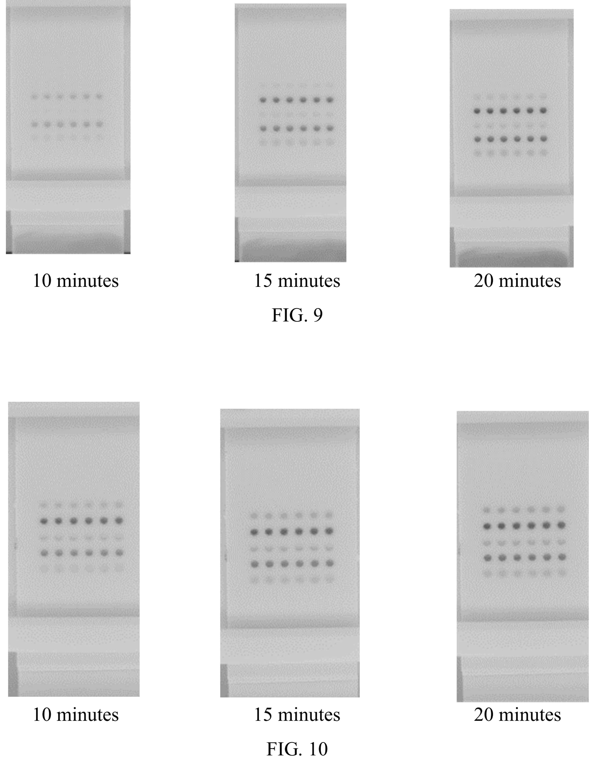

The results are as shown in FIG. 9 (Mode 1) and FIG. 10 (Mode 2), showing that the signals of various sites in the chip reaction area in Mode 2 can reach a large value in a short time because such sites simultaneously contact with the substrate. Mode 1 shows substantially obvious results in 15 minutes, and Mode 2 shows good testing results in 10 minutes.

II. The addition of the reaction liquid into the reaction area will cause the liquid outlet end not to be affected by the signal intensity of the liquid inlet end.

The changes in the signal intensities of the protein binding sites where the reaction liquid is added into the liquid sample contact area 202 and directly added onto the reaction area are compared to illustrate that the signal intensity of the immune complex at the same channel at the liquid outlet end cannot be affected by the signal intensity of the immune complex at the liquid inlet end.

A micro-applicator (Hangzhou Bufeng Technology Co., Ltd.) was used to drip specific antigens of CMV, HSV1, HSV2, RV and TOX (purchased from Fapon Biotech Inc.) onto the porous membrane of the immune reaction chip (the nitrocellulose membrane was used here, where the pore size of the membrane was 12µm, the width of the membrane was 13mm, and the thickness of the liquid blocking element was 0.8mm), the concentrations thereof were 0.2mg/mL, 0.2mg/mL, 0.05mg/mL, 0.2mg/mL, 0.05mg/mL and 50nL/site respectively; and such specific antigens were dried for 2 hours at 45°C. The membrane of the immobilized antigen and the water absorption element were together loaded into a plastic shell. A solid-phase chip reaction unit including a liquid sample contact area, a reaction area and a liquid discharge area was prepared.

For the convenience of expression, the liquid involved in the immune reaction was numbered by Arabic numerals:
Liquid #1: a recombinant specific anti-CAM IgM antibody, a recombinant specific anti-HSV1 IgM antibody, a recombinant specific anti-HSV2 IgM antibody, a recombinant specific anti-RV IgM antibody, and a recombinant specific anti-TOX IgM antibody (purchased from Fapon Biotech Inc.) were diluted with a pH7.4 phosphate buffer including 10mg/mL OsrHSA and 10mg/mL Tween 20.
Liquid #1 - 1: only a TOX IgM antibody was diluted by 8000 times (under existing conditions, the observable signal at this concentration was weak), and the rest antibodies were not diluted (under the existing conditions, the observable signal at this concentration was extremely weak or absent).
Liquid #1 - 2: only the TOX IgM antibody was diluted by 8000 times (under existing conditions, the observable signal at this concentration was weak), and the rest antibodies were diluted by 500 times (under the existing conditions, the observable signal at this concentration was extremely weak or absent)
Liquid #2: pH7.4 phosphate buffer including 10mg/mL casein.
Liquid #3: pH7.4 phosphate buffer including 10mg/mL casein.
Liquid #4: enzyme working solution including a specific anti-human IgM-HRP (horseradish peroxidase) conjugate.
Liquid #5: pH7.4 phosphate buffer including 1mg/mL Tween 20
Liquid #6: pH7.4 phosphate buffer including 1mg/mL Tween 20
Liquid #7: reaction liquid (Hangzhou Bufeng Technology Co., Ltd.) including 3,3',5,5'-tetramethylbenzidine.

The liquid sample contact area of the solid-phase chip reaction unit was sequentially in contact with liquid #1, liquid #2, liquid #3, liquid #4, liquid #5, and liquid 6# for 5 minutes, respectively. After contacting with the liquid sample contact area, different liquids diffused through the reaction area of the solid-phase chip reaction unit through microporous channels on the nitrocellulose membrane via the surface tension between the liquids and the microporous channels and the capillary driving force of the liquid absorption element on the liquid discharge area.

After liquid #6 ended up its contact, the liquid sample contact area contacted with 400µL of liquid #7 (Mode 1) or 200µL of TMB liquid #7 was directly added into the reaction area (Mode 2); then, in Mode 1, the intensities of TOX IgM detection signals of liquid #1 - 1 and liquid #1 - 2 were compared at 25 minutes; in Mode 2, the intensities of TOX IgM detection signals of liquid #1 - 1 and liquid #1 - 2 were compared at 10 minutes.

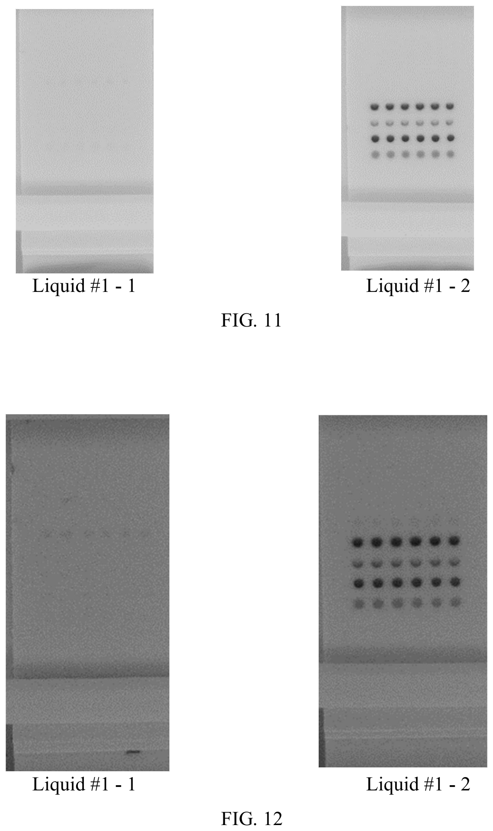

The results are as shown in FIG. 11 and FIG. 12. The detection results of TOX IgM located at the uppermost (namely, the liquid outlet end) are affected by those at the liquid inlet end to the greatest extent, but not excluded from the scope of the present invention, and this is also an embodiment. The intensity of the observable signal formed by the immune complex = [specific anti-TOX IgM concentration ]× [specific anti-human IgM-HRP concentration ]×[TMB-H2O2 concentration]. In the embodiment, the concentration of the specific anti-human IgM-HRP in liquid #4 and the concentration of TMB-H2O2 in a TMB substrate solution both approach to be excessive. The comparison shows that the detection results of TOX IgM in Mode 2 are not affected by those at the liquid inlet end.

### Embodiment 4: Canine allergen testing device.

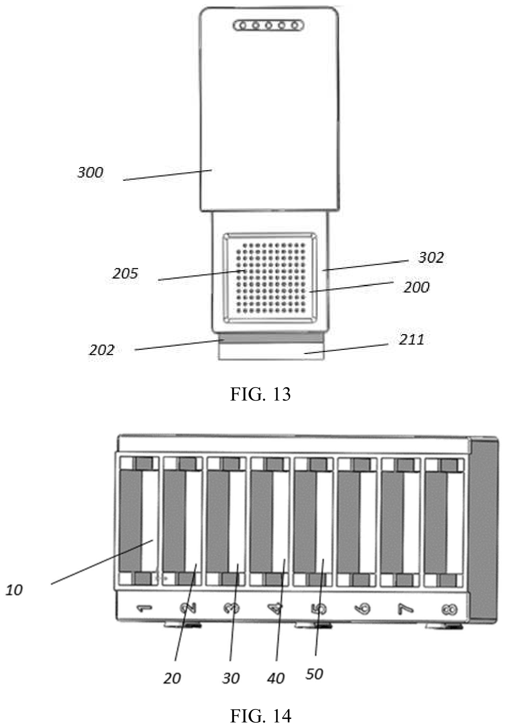

The devices and the combinations of solutions and reagents as shown in FIG. 13 and FIG. 14 were provided, where the testing device was provided with a porous membrane (a specific structure was introduced), and a solution reagent kit was filled with necessary reagents.

### Preparation of solid-phase reaction unit:

Mouse anti-goat monoclonal antibody (MAG), an anti-canine IgE monoclonal antibody (anti-canine IgE Mab) and 74 allergenic substances are immobilized onto the designated locations of the membrane (the nitrocellulose membrane was used here, where the pore size of the membrane was 10µm, the width of the membrane was 26 mm, and the thickness of the liquid blocking element was 0.8mm). The drop concentration of various substances was controlled at the range of 100ng/mL-1.0mg/mL; and the diluent was a phosphate buffer including a red water-soluble dye (50nL/site), and dried for 2 hours at 45°C to immobilize the substances on the membrane.

The membrane and the water absorption element were together loaded into a plastic shell and immobilized (as shown in FIG. 6 and FIG. 13). A solid-phase chip reaction unit including a liquid sample contact area, a reaction area and a liquid discharge area was prepared.

The specific arrangement of liquid drops on the porous membrane is as shown in the following table (the testing result control units are located at a first row and a first left column):

| | MAG | MAG | MAG | MAG | MAG | MAG | MAG | MAG | MAG |
|---|---|---|---|---|---|---|---|---|---|
| MAG | Candida albicans | Penicill ium | Aspergillus fumigatus | Alternaria alternata | Bacillomycin | Tyrophagus putrescentiae | Dermatoph agoides pteronyssin us | Blomia tropicalis | Psoriasis |
| MAG | Duck feather | Cat dander | Wool | Sheep dander | Amaranthus spinosus | Poa pratensis | Taraxacum | Populus | Chenopodi um quinoa Willd |
| MAG | Willow | Pine | Bermuda grass | Birch | Elm | Paper mulberry | Firmiana simplex | Mango pollen | Rumex japonicus Houtt. |
| MAG | Humulus japonicus | Black ant | Flea | Mosquito | Cockroach | Salmon | Sardine | Tuna | Anchovy |
| MAG | Mackerel | Crab | Shrimp | Cod | Lateolabrax japonicus | Soybean | Bread yeast | Beer yeast | Peanut |
| MAG | Rice | Wheat | Corn | Kiwi fruit | Potato | Pear | Pineapple | Sweet potato | Watermelo n |
| MAG | Banana | Spinach | Carrot | Broccoli | Duck | Beef | Mutton | Whole egg | Milk |
| MAG | Chicken | Pork | Venison | Turkey meat | | | | | |
| MAG | | | | | | | | | |
| Anti-canine IgE | | | | | | | | | |

The chip reaction area after preparation is as shown in FIG. 13.

### Preparation of liquid-phase reaction unit;

The following liquids were in advance added into a porous groove (FIG. 15) for liquid-phase reaction:
A sample diluent, namely a pH7.4 phosphate buffer including Triton-X100, was in advance added into well 1 with 150µL/well.

A blocking liquid, namely a pH7.4 phosphate buffer including Casein-Na, bovine serum, and Triton-X100, was in advance added into well 2 with 300µL/well.

An enzyme conjugate solution, namely an enzyme working solution including goat anti-canine IgE-HRP, was in advance added into well 3 with 300µL/well.

A washing liquid, namely a pH7.4 phosphate buffer including Tween 20, was in advance added into well 4 with 300µL/well.

A washing liquid, namely a pH7.4 phosphate buffer including Tween 20, was in advance added into well 5 with 300µL/well.

The porous groove was in advance filled with liquids, and thermally sealed by an aluminum film, such that the above liquids were protected in each well of the porous groove.

Reaction liquid: 500µL of reaction liquid was stored in a plastic bottle and sealed for later use.

### Determination method of IgE in canine serum:

A liquid-phase unit was taken and laid flat on a workbench, and the porous groove was tapped to ensure that all liquid reagents preloaded return to the bottom of the porous groove. The aluminum film was pulled away along the direction of the upper surface of the porous groove until all the liquids were exposed. 150µL of canine serum was taken and added into the well 1, and then repeatedly blown, such that the sample and the sample diluent were mixed well.

The liquid sample contact area 202 of the solid-phase unit was inserted into the well 1 and left standing; in this case, the liquid in the well 1 would automatically diffuse from bottom to top, flow into the porous membrane, and flow through the testing area.

After 7 minutes, the solid-phase unit was taken out of the well 1 and moved over the well 2; the liquid sample contact area 202 of the solid-phase unit was inserted into the well 2 and left standing; and in this case, the liquid in the well 2 would automatically diffuse from bottom to top.

After 7 minutes, the solid-phase unit was taken out of the well 2 and moved over the well 3; the liquid sample contact area of the solid-phase unit was inserted into the well 3 and left standing; and in this case, the liquid in the well 3 would automatically diffuse from bottom to top.

After 7 minutes, the solid-phase unit was taken out of the well 3 and moved over the well 4; the liquid sample contact area of the solid-phase unit was inserted into the well 4 and left standing; and in this case, the liquid in the well 4 would automatically diffuse from bottom to top.

After 7 minutes, the solid-phase unit was taken out of the well 4 and moved over the well 5; the liquid sample contact area of the solid-phase unit was inserted into the well 5 and left standing; and in this case, the liquid in the well 5 would automatically diffuse from bottom to top.

After 7 minutes, the solid-phase unit was taken out of the well 5 and laid flat on the workbench; the reaction liquid was poured into the chip window of the shell; in this case, a substrate solution was laid flat in the reaction area in the window and slowly diffused to a water absorption area through the membrane below the liquid blocking element. After 10 minutes, the substrate solution temporarily staying in the reaction area would be completely absorbed by the water absorption area; and whether locations, total IgE sites, and specific IgE spots are present in the reaction area was observed within 5 minutes.

The locations were distributed in a rectangular coordinate. Horizontal and vertical locations were compared to confirm the spot location, and the spot location was corresponding to the specific allergenic substance making immune reaction. This judges the presence or absence of specific IgE binding to the substances in the serum. The total IgE sites are located at the bottom of the longitudinal location, and not on a same diffusion path as the site of the allergenic substance, so tests of various specific IgE sites are not affected.

FIG. 5B shows testing results of a canine serum sample. Such testing results show that the canine serum sample includes specific IgE that reacts with allergenic substances such as aspergillus fumigatus, tyrophagus putrescentiae, wool, sheep dander, cockroaches, anchovies, potatoes and watermelons, and the total IgE in the liquid sample is high.

For example, the above operations can be manually performed during home test. Of course, such operations can be automatically performed by equipment, such as tearing off a sealing membrane of a solution tank, adding the sample into a No. 1 solution chamber, inserting the liquid sample contact area 202 of the test strip into a No. 1 tank, and placing the No. 1 tank into the equipment. The equipment has a structure similar to a manipulator to clamp the testing device to the absorbent paper, and then time counting starts to be done. After a specific time, the test strip is taken out of the No. 1 tank and sequentially inserted into subsequent solution chambers for reaction. After the last well ends up its contact, the reaction liquid can be taken and directly dripped; alternatively, the reaction liquid can be injected into the well 6# to allow the reaction liquid to flow from the porous membrane to the testing area. Such equipment can be used to implement continuous treatment in a laboratory. After the reaction, reading equipment can be automatically configured, for example, a camera unit can be configured to directly photograph the testing area, directly process images, directly output the testing results, and of course, the testing results of the testing unit can also be observed with naked eyes.

### Embodiment 6: Influence of blocking reagent on testing results

The specific implementations in Embodiment 6 are the same as those in Embodiment 5. With a liquid sample being a negative sample, there are no color spots in the testing area if a blocking reagent is used (for example, a reagent in well 2), and there are color spots in the testing area if no blocking reagent is used. This indicates that each testing unit has non-specific sites and the background appears in the blank area. Goat anti-canine IgE-HRP enzymes can be captured and a false positive result is generated. In addition, color precipitates between testing sites, making the background not very clean and clear. Therefore, when multiple allergens are tested, the blocking liquid can eliminate nonspecific binding, avoid false positive results and make the test background clean, thereby reducing the impact of the background on the testing area.

## Claims

1. A device for testing an allergen antibody in a liquid sample, comprising :
a testing area and a water absorption area,
wherein the testing area is used to contact with liquid, and the water absorption area is located downstream of the testing area and used to absorb the liquid from the testing area, such that the liquid is allowed to flow laterally through the testing area and then flow onto the water absorption area; and wherein a plurality of different first receptors specifically binding to the allergen antibody in the liquid sample are fixed onto the testing area.

2. The device according to claim 1, wherein the testing area is located on a porous membrane.

3. The device according to claim 2, wherein one end of the porous membrane is used to contact with the liquid and the other end thereof is used to contact with the water absorption area, such that the water absorption area is allowed to absorb the liquid on the porous membrane, and the liquid is allowed to flow from one end of the porous membrane contacting with the liquid to the other end of the porous membrane contacting with the water absorption area through the testing area.

4. The device according to claim 3, wherein the liquid comprises at least two or more of a liquid for dissolving a liquid sample, a liquid including a label, a washing liquid, a blocking liquid, and a reaction liquid.

5. The device according to claim 4, wherein the liquid including a label comprises a plurality of different second receptors that include the label and specifically bind to the allergen antibody (IgE), and the second receptors are capable to specifically bind to the allergen antibody (IgE) or a complex formed by the allergen antibody (IgE) and the first receptors.

6. The device according to claim 5, wherein the second receptors comprise an antibody against the allergen antibody (IgE), and the first receptors comprise an allergen or the antibody against the allergen antibody (IgE).

7. The device according to claim 6, wherein each of the second receptors carries a same label.

8. The device according to claim 7, wherein the label comprises enzyme, colored particles or dyes, or a fluorescent label.

9. The device according to claim 8, wherein the label is the enzyme.

10. The device according to claim 9, wherein one end of the porous membrane contacting with the liquid is capable to continuously contact with at least two or more of the liquid sample, the liquid including a label, the washing liquid, a liquid containing a blocking reagent, and the reaction liquid; alternatively, one end of the porous membrane contacting with the liquid is capable to continuously and sequentially contact with the liquid sample, a first washing liquid, a liquid containing a blocking reagent, the liquid including a label, a second washing liquid, and the reaction liquid.

11. The device according to claim 10, further comprising a liquid blocking element, wherein the liquid blocking element enables the reaction liquid to stay in the testing area of the porous membrane or enables the reaction liquid not to flow from the surface of the porous membrane.

12. The device according to claim 11, wherein the liquid blocking element is located between the testing area of the porous membrane and the end of the porous membrane contacting with the liquid; or/and, the liquid blocking element is located between the testing area and the water absorption area; or/and, the liquid blocking element covers the porous membrane.

13. The device according to claim 12, wherein the reaction liquid comprises a reaction reagent that is capable to react with the enzyme to generate a readable signal.

14. The device according to claim 11, wherein the liquid blocking element is located around an allergen array in the testing area, such that the reaction liquid is capable to stay in an area where the allergen array is fixed.

15. The device according to claim 11, wherein the liquid blocking element is located on the porous membrane on which a liquid pool area is enclosed; the testing area is located in an area enclosed by the liquid blocking element; and the reaction liquid is capable to stay in the liquid pool.

16. The device according to claim 11, wherein the liquid blocking element does not substantially affect the capillary force of the porous membrane, such that the water absorption area is capable to help the reaction liquid located on the porous membrane flow into the water absorption area.

17. The device according to claim 11, wherein the liquid blocking element is substantially composed of hydrophobic polymer materials, and the hydrophobic polymer materials comprise one or more of polyvinyl chloride, polyester, polyurethane, and polystyrene.

18. The device according to claim 4, wherein the liquid for dissolving a liquid sample does not contain the blocking reagent.

19. The device according to claim 18, wherein the blocking reagent is provided to block a porous and/or non-specific site of a blank area on the porous membrane.

20. The device according to claim 18, wherein the blocking reagent comprises casein.

21. The device according to claim 2, wherein the porous membrane comprises a nitrocellulose membrane or a polyvinylidene fluoride membrane.

22. The device according to claim 3,wherein the porous membrane is that when using pure water as liquid to take less than 5 minutes to diffuse 4cm along thereof.

23. The device according to claim 1, wherein the water absorption area comprises an absorbent pad; the absorbent pad comprises hydrophilic fiber materials; and the hydrophilic fiber materials are selected from natural plant fibers or chemically synthesized acetate fibers.

24. The device according to claim 1, wherein the liquid sample is a serum, plasma or whole blood sample.

25. The device according to claim 1, further comprising a test reference area for testing the allergen antibody in a total sample.

26. The device according to claim 1, wherein the first receptors are fixed onto the testing area in an array.

27. A method for testing an allergen antibody in a liquid sample, comprising:
providing a testing device, wherein the testing device comprises a testing area and a water absorption area, a plurality of first receptors are fixed onto the testing area in an array, and each of the first receptors is capable to specifically bind to an allergen antibody in the liquid sample;
the testing area is located on a porous membrane, wherein the porous membrane is provided with a first end and a second end, the water absorption area is connected with the first end of the porous membrane, and the second end of the porous membrane is allowed to contact with the liquid sample, such that the liquid sample is allowed to flow through the testing area of the porous membrane and then flows into the water absorption area.

28. The method according to claim 27, wherein the second end of the porous membrane is allowed to contact with a blocking liquid containing a blocking reagent, and the blocking reagent is allowed to fill a blank area on the porous membrane or block a non-specific binding site.

29. The method according to claim 28, wherein the second end of the porous membrane is allowed to contact with a plurality of second receptors containing a label, and each of the second receptors is capable to specifically bind to the allergen antibody in the liquid sample, such that the second receptors containing a label flow into the water absorption area through the testing area under the capillary action of the water absorption area.

30. The method according to claim 29, wherein the porous membrane further comprises an area formed by a liquid blocking element, the area comprises the testing area therein, and the liquid blocking element enables a reaction liquid to stay in the testing area.

31. The method according to claim 30, wherein the reaction liquid is directly applied to the testing area, such that the liquid blocking element enables the reaction liquid to stay in the testing area.

32. The method according to claim 31, wherein the reaction liquid comprises a reaction reagent, and the reaction reagent is capable to react with the label to generate signals, and the presence or absence or quantity of analytes is obtained according to the presence or absence or quantity of the signals.

33. The method according to claim 32, wherein the signals are readable and preferably read by naked eyes.

34. The method according to claim 31, wherein when the reaction liquid stays in the testing area, the water absorption area is capable to absorb part of the reaction liquid that is located on the porous membrane and flows onto the water absorption area.

35. The method according to claim 34, wherein the reaction liquid is allowed to stay in the testing area for 10 seconds to 60 minutes.

36. The method according to claim 27, wherein after the second end of the porous membrane is allowed to contact with the liquid sample, the first end of the porous membrane is allowed to contact with a washing liquid and then a liquid including a label.
